# EUROPEAN PATENT APPLICATION

(11) **EP 4 431 129 A1**
(43) Date of publication of application: **18.09.2024**
(21) Application number: 22893050.9
(22) Date of filing: 18.10.2022
(51) Int. Cl.: A61M 5/142, A61M 5/168, A61M 5/145, A61M 5/148

(54) **MEDICINAL SOLUTION DISCHARGE ASSEMBLY AND MEDICINAL SOLUTION INJECTION DEVICE INCLUDING SAME**

(30) Priority: 12.11.2021 KR 20210155352; 17.12.2021 KR 20210181552
(71) Applicant: Eoflow Co., Ltd., Seongnam-si, Gyeonggi-do 13605 (KR)
(72) Inventor: HAN, Yong Joon, Seoul 05236 (KR); KIM, Seung Ha, Goyang-si Gyeonggi-do 10558 (KR); HAN, Sang Hyun, Incheon 21549 (KR); ROH, Hyun Duk, Seoul 03690 (KR); MIN, Kyoung In, Gimpo-si Gyeonggi-do 10129 (KR); JU, Sang A, Anyang-si Gyeonggi-do 13903 (KR); KIM, Jesse Jaejin, Seongnam-si, Gyeonggi-do 13562 (KR)
(74) Representative: AWA Sweden AB
(86) International application number: PCT/KR2022/015806
(87) International publication number: WO 2023/085627

(57) **Abstract**

The present disclosure provides a medical liquid discharge assembly including a driving part configured to move in a preset direction, a rotating part configured to come into contact with the driving part, and to rotate in one direction in response to the movement of the driving part, a tube part disposed adjacent to the rotating part and including a flexible material, a pressure-applying part configured to come into contact with the rotating part, and to come into contact with and apply pressure to the tube part in response to the rotation of the rotating part, and a locking part configured to move in a preset direction, and to fix a position of the pressure-applying part by coming into contact with and supporting one surface of the pressure-applying part while the pressure-applying part applies pressure to the tube part.

## Description

### Technical Field

Embodiments of the present disclosure relate to a medical liquid discharge assembly and a medical liquid injection device including the same.

### Background Art

In general, medical liquid injection devices such as insulin injection devices are used to inject medical liquids into patients' bodies. Such medical liquid injection devices are used by professional medical staff such as nurses or doctors but are mostly used by ordinary persons such as caretakers or patients themselves.

In the case of diabetic patients, particularly pediatric diabetic patients, it is necessary to inject medical liquids such as insulin into a human body at regular intervals. A patch-type medical liquid injection device that may be used by being attached to a human body for a predetermined period of time has been developed, and such a medical liquid injection device may be used while being attached as a patch type to the abdomen or waist of a patient for a predetermined period of time.

In order to increase the effect of the medical liquid injection, the medical liquid injection device needs to be controlled to precisely inject the medical liquid into the body of a patient, and accordingly, it is important to precisely inject a small amount of the medical liquid through a small-sized medical liquid injection device.

The medical liquid injection device attached to the human body needs to be comfortable to wear, to be convenient to use, to be durable, and to be driven with low power. In particular, since a patient directly attaches the medical liquid injection device to the skin and uses it, it is important for a user to drive the medical liquid injection device conveniently and safely.

It is important for a medical liquid injection device to inject a set amount of a medical liquid, and thus, in order to inject the medical liquid in a set amount, a device that distributes a set amount of medical liquid is required. Research is continuously being conducted to distribute and discharge a medical liquid in a set amount using mechanical methods.

### Disclosure

### Technical Problem

The present disclosure provides a medical liquid discharge assembly and a medical liquid injection device including the same, capable of improving the stability of medical liquid injection by allowing a locking part to fix the position of a pressure-applying part by coming into contact with and supporting the pressure-applying part, so that a tube part remains in a compressed state.

The present disclosure also provides a medical liquid discharge assembly and a medical liquid injection device including the same, capable of improving the stability of medical liquid injection by allowing a locking part to fix the position of a pressure-applying part by coming into contact with and supporting the pressure-applying part, so that a tube part remains in a compressed state.

### Technical Solution

According to an aspect of the present disclosure, there is provided a medical liquid discharge assembly including a driving part configured to move in a preset direction, a rotating part configured to come into contact with the driving part and to rotate in one direction in response to the movement of the driving part, a tube part disposed adjacent to the rotating part and including a flexible material, a pressure-applying part configured to come into contact with the rotating part, and to come into contact with and apply pressure to the tube part in response to the rotation of the rotating part, and a locking part configured to move in a preset direction, and to fix a position of the pressure-applying part by coming into contact with and supporting one surface of the pressure-applying part while the pressure-applying part applies pressure to the tube part.

Further, the pressure-applying part may apply pressure to the tube part so that the tube part is compressed at a contact point at which the pressure-applying part comes into contact with the tube part.

Further, at least a portion of the tube part may have a curved section extending in a circumferential direction.

Further, the pressure-applying part may rotate while applying pressure to the tube part.

Further, the pressure-applying part may include a pressure-applying body configured to move in a preset direction on the rotating part and to come into contact with and apply pressure to the tube part, and a pushing part disposed between the pressure-applying body and the rotating part, and configured to push the pressure-applying body in such a direction that the pressure-applying body is spaced apart from the rotating part.

Further, the pushing part may be provided as a plurality of pushing parts.

Further, the pushing part may exhibit an elastic restoring force in such a direction that the pushing part is away from the rotating part.

Further, the locking part may be formed of an elastically deformable material.

Further, one end portion of the locking part may exhibit an elastic restoring force in a direction toward a longitudinal central axis of the locking part.

According to an aspect of the present disclosure, there is provided a medical liquid injection device including a needle assembly, a reservoir configured to store a medical liquid to be discharged to the needle assembly, a driving part configured to move in a preset direction, a rotating part configured to come into contact with the driving part, and to rotate in one direction in response to the movement of the driving part, a tube part disposed between the needle assembly and the reservoir and including a flexible material, a pressure-applying part configured to come into contact with the rotating part, and to come into contact with and apply pressure to the tube part in response to the rotation of the rotating part, and a locking part configured to move in a preset direction, and to fix a position of the pressure-applying part by coming into contact with and supporting one surface of the pressure-applying part while the pressure-applying part applies pressure to the tube part.

According to another aspect of the present disclosure, there is provided a medical liquid discharge assembly including a driving part configured to move in a preset direction, a rotating part configured to come into contact with the driving part, and to rotate in one direction in response to the movement of the driving part, a tube part formed of a flexible material, a pressure-applying part disposed to face the rotating part with the tube part interposed therebetween, and configured move in a direction of a first axis and to come into contact with and apply pressure to the tube part, and a locking part disposed to be in contact with the pressure-applying part, configured to move in a direction of a second axis, which forms a certain angle with the first axis, and including a locking body located on a movement path of the pressure-applying part.

Further, the locking part may include a locking base part disposed to be spaced apart from the pressure-applying part, and the locking body configured to move in the direction of the second axis on the locking base part.

Further, the rotating part may include a rotating body configured to rotate upon receiving a driving force from the driving part; and a contact part disposed on the rotating body and configured to come into contact with the tube part.

Further, the contact part may be rotatably installed on the rotating body.

According to another aspect of the present disclosure, there is provided a medical liquid injection device including a needle assembly, a reservoir configured to store a medical liquid to be discharged to the needle assembly, a driving part configured to move in a preset direction, a rotating part configured to come into contact with the driving part, and to rotate in one direction in response to the movement of the driving part, a tube part disposed between the needle assembly and the reservoir and including a flexible material, a pressure-applying part disposed to face the rotating part with the tube part interposed therebetween, and configured move in a direction of a first axis and to come into contact with and apply pressure to the tube part, and a locking part disposed to be in contact with the pressure-applying part, configured to move in a direction of a second axis, which forms a certain angle with the first axis, and including a locking body located on a movement path of the pressure-applying part.

Other aspects, features, and advantages other than those described above will become apparent from the following drawings, claims, and detailed description of the disclosure.

### Advantageous Effects

According to an embodiment of the present disclosure, in a medical liquid discharge assembly and a medical liquid injection device including the same, a locking part can fix the position of a pressure-applying part by coming into contact with and supporting the pressure-applying part, so that a tube part can remain in a compressed state, thereby improving the stability of medical liquid injection.

Further, since the tube part remains in a compressed state, it is possible to prevent under-injection of medical liquid, reflux of medical liquid, or the like, thereby improving the stability of medical liquid injections.

Further, since the position of the pressure-applying part is fixed, it is possible to maintain a state in which an internal cross-sectional area of the tube part is zero, and to prevent deformation in the degree of compression of the tube part due to an elastic restoring force thereof.

Further, it is possible to block the movement of the pressure-applying part by allowing a locking part to move along a second axis, which forms a certain angle with a first axis that is a moving axis of the pressure-applying part, and to be located on a movement path of the pressure-applying part.

Further, since the movement of the pressure-applying part is blocked, it is possible to maintain the state in which the pressure-applying part is in contact with and compresses the tube part.

In addition, by maintaining the compressed state of the tube part, a set amount of medical liquid can be consistently discharged through a needle assembly from an interior of the tube part.

Further, a structure, in which the locking part moves linearly along the second axis forming a certain angle with the first axis, so that the locking part is disposed on a movement path of the pressure-applying part moving in the direction of the first axis, allows an interior space of the medical liquid injection device to be utilized efficiently and allows a structure that blocks the movement of the pressure-applying part to be simplified.

Of course, the scope of the present disclosure is not limited by these effects.

### Description of Drawings

FIG. 1 is a perspective view illustrating a medical liquid injection device according to embodiments of the present disclosure.
FIG. 2 is a plan view illustrating an interior of the medical liquid injection device according to an embodiment of the present disclosure.
FIG. 3 is a bottom view illustrating a bottom surface of a body part according to an embodiment of the present disclosure.
FIG. 4 is a view partially illustrating a medical liquid discharge assembly according to an embodiment of the present disclosure.
FIGS. 5A to 5C are views partially illustrating an operation process of the medical liquid discharge assembly according to an embodiment of the present disclosure.
FIGS. 6A and 6B are views partially illustrating an operation process of a locking part according to an embodiment of the present disclosure.
FIG. 7 is a perspective view illustrating a medical liquid discharge assembly according to another embodiment of the present disclosure.
FIG. 8 is a perspective view illustrating a rotating part and a pressure-applying part according to another embodiment of the present disclosure.
FIG. 9 is an exploded perspective view illustrating the rotating part and the pressure-applying part according to another embodiment of the present disclosure.
FIGS. 10A to 10C are views illustrating an operation process of the medical liquid discharge assembly according to another embodiment of the present disclosure.
FIG. 11A is a cross-sectional view taken along line I-I of FIG. 10A.
FIG. 11B is a cross-sectional view taken along line II-II of FIG. 10C.
FIG. 12 is a view partially illustrating the state of FIG. 10A.
FIG. 13 is a view partially illustrating the state of FIG. 10C.
FIG. 14 is a perspective view illustrating a medical liquid injection device according to an embodiment of the present disclosure.
FIG. 15 is a plan view illustrating an interior of the medical liquid injection device according to an embodiment of the present disclosure.
FIGS. 16 and 17 are enlarged views partially illustrating a locking part, a rotating part, and a pressure-applying part according to an embodiment of the present disclosure.
FIG. 18 is a block diagram illustrating a control part according to an embodiment of the present disclosure.
FIG. 19 is a plan view illustrating an interior of a medical liquid injection device according to another embodiment of the present disclosure.
FIGS. 20 and 21 are perspective views schematically illustrating a locking part and a pressure-applying part according to another embodiment of the present disclosure.
FIGS. 22 and 23 are enlarged views partially illustrating the locking part, a rotating part, and the pressure-applying part according to another embodiment of the present disclosure.

### Mode for Invention

While the present disclosure is susceptible to various modifications and alternative forms, specific embodiments thereof are shown by way of example in the drawings and will herein be described in detail. The effects and features of the present disclosure and the accompanying methods thereof will become apparent from the following description of the embodiments, taken in conjunction with the accompanying drawings. However, the present disclosure is not limited to the embodiments disclosed below but may be implemented in various forms.

Hereinafter, the embodiments of the present disclosure will be described below in detail with reference to the accompanying drawings, and when the embodiments of the present disclosure are described with reference to the drawings, the same or corresponding components are given the same reference numerals, and repetitive descriptions thereof will be omitted.

In the following embodiments, singular forms are intended to include plural forms as well, unless the context clearly indicates otherwise.

In the following embodiments, the terms such as "including," "having," and "comprising" are intended to indicate the existence of features or components disclosed in the specification, and are not intended to preclude the possibility that one or more other features or components may be added.

When a certain embodiment may be implemented differently, a specific process sequence may be performed differently from the described order. For example, two consecutively described processes may be performed substantially at the same time or performed in an order opposite to the described order.

In the drawings, for convenience of description, sizes of components may be exaggerated or reduced. For example, since the size and thickness of each component shown in the drawings are arbitrarily indicated for convenience of description, the following embodiment is not necessarily limited to what is illustrated.

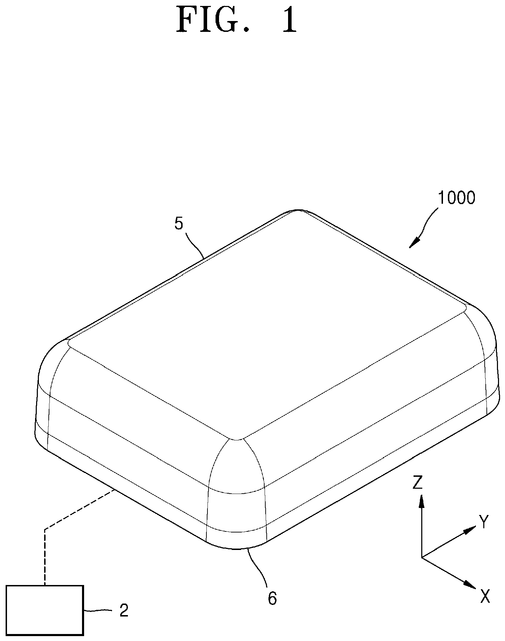

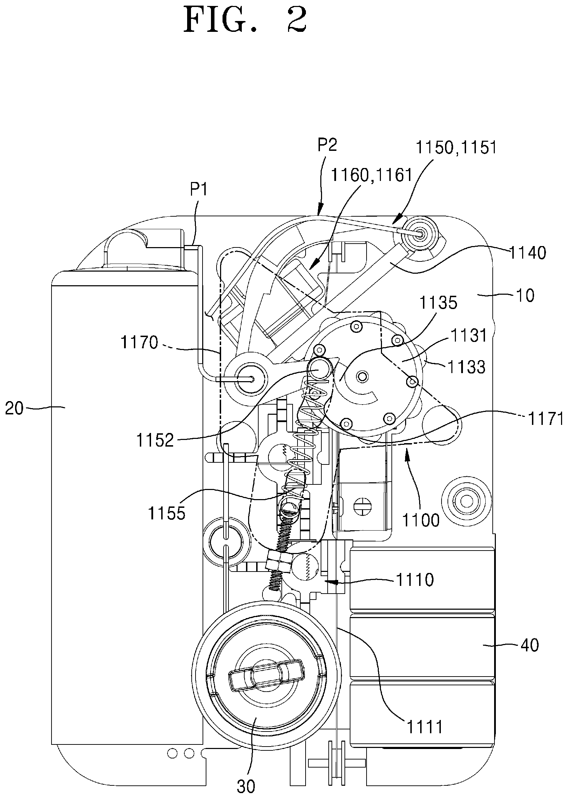

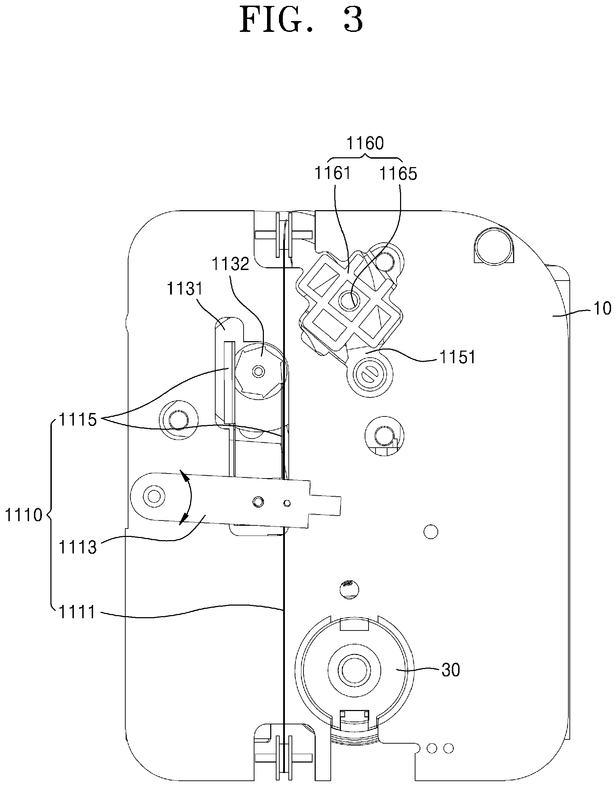

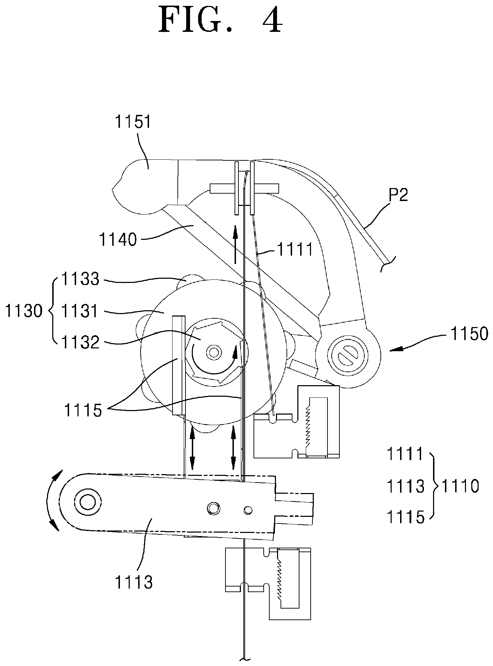

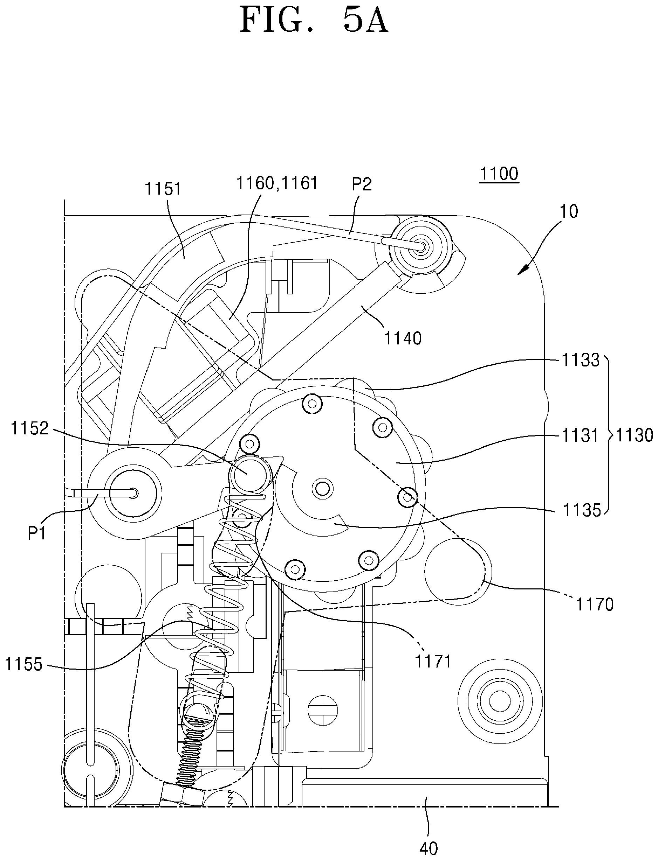

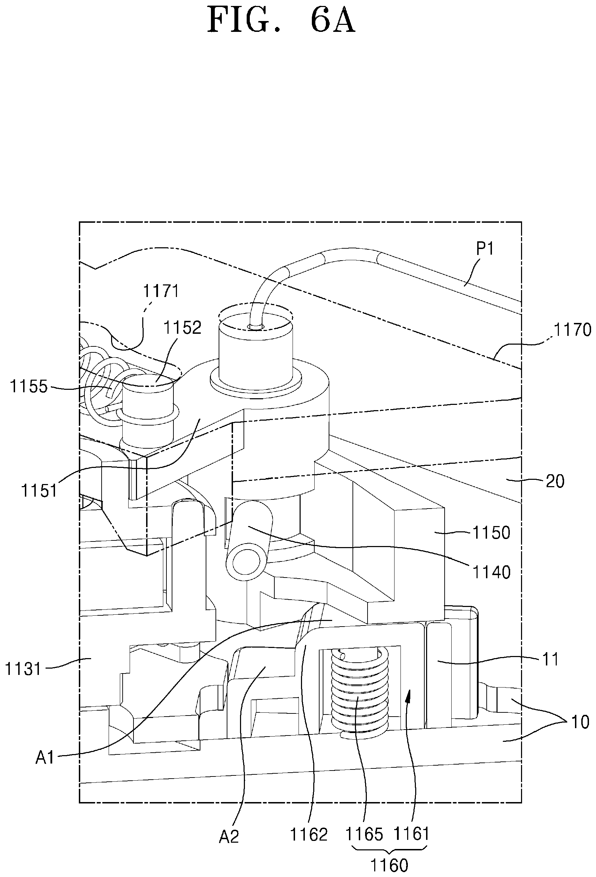

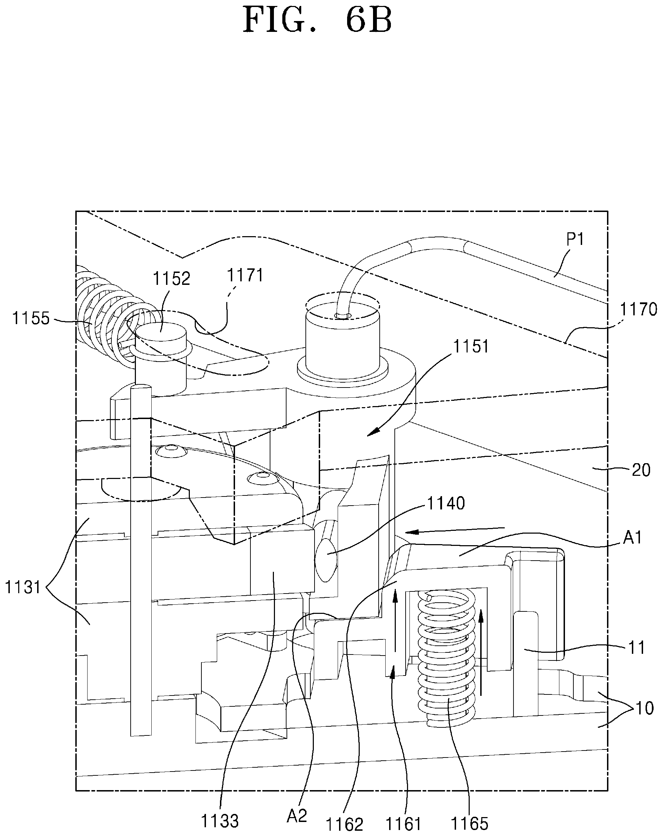

FIG. 1 is a perspective view illustrating a medical liquid injection device according to embodiments of the present disclosure. FIG. 2 is a plan view illustrating an interior of the medical liquid injection device according to an embodiment of the present disclosure. FIG. 3 is a bottom view illustrating a bottom surface of a body part according to an embodiment of the present disclosure. FIG. 4 is a view partially illustrating a medical liquid discharge assembly according to an embodiment of the present disclosure. FIGS. 5A to 5C are views partially illustrating an operation process of the medical liquid discharge assembly according to an embodiment of the present disclosure. FIGS. 6A and 6B are views partially illustrating an operation process of a locking part according to an embodiment of the present disclosure.

Referring to FIG. 1, a medical liquid injection device 1000 according to an embodiment of the present disclosure is attached to a medical liquid injection subject, and may inject a preset amount of medical liquid stored therein to a user.

In an optional embodiment, the medical liquid injection device 1000 may be mounted on the body of the medical liquid injection subject (hereinafter referred to as the "user").

In an optional embodiment, the medical liquid injection device 1000 may also be mounted on an animal and may inject a medical liquid thereto.

The medical liquid injection device 1000 according to an embodiment of the present disclosure may be used for various purposes depending on the type of medical liquid to be injected. For example, the medical liquid may include an insulin-based medical liquid for a diabetic patient, and may include a medical liquid for other pancreas, a medical liquid for heart, and other various types of medical liquids.

Referring to FIG. 1, the medical liquid injection device 1000 may be connected to a remote device 2 that is wired or wirelessly connected.

A user may manipulate the remote device 2 to use the medical liquid injection device 1000 and monitor the state of use of the medical liquid injection device 1000. For example, the amount of medical liquid injected from the medical liquid injection device 1000, the number of injections of the medical liquid, the amount of medical liquid stored in a reservoir 20, user's bio information, and the like may be monitored, and based on this, the user may drive the medical liquid injection device 1000.

The remote device 2 according to an embodiment of the present disclosure can remotely transmit and receive signals to and from a control module (not shown in the drawings) provided in the medical liquid injection device 1000, and the control module may receive signals from the external remote device 2 and apply an electric signal to a driving part 1110 to be described later.

The driving part 1110, upon receiving an electrical signal from the control module, may transmit a driving force to a rotating part 1130, which will be described later, to rotationally drive the rotating part 1130, and may rotate the rotating part 1130 in a preset direction (in a counterclockwise direction based on FIG. 4).

In the present specification, the term "remote device 2" refers to a communication terminal that may use an application in a wired or wireless communication environment. Here, the remote device 2 may be a user's portable terminal.

In more detail, the remote device 2 may include a computer (e.g., desktop, laptop, tablet, or the like), a media computing platform (e.g., cable, satellite set-top box, digital video recorder), a handheld computing device (e.g., personal digital assistant (PDA), e-mail client, or the like), a mobile phone in any form, a form of a wearable device that can be attached or mounted on the user's body, or any form of another kind of computing or communication platform, but the present disclosure is not limited thereto.

The medical liquid injection device 1000 and the remote device 2 may communicate through a communication network. In this case, the communication network refers to a communication network providing an access path so that the remote device 2 may transmit and receive data after accessing a service server (not shown in the drawings). The communication network may be, for example, a wired network such as local area networks (LANs), wired area networks (WANs), metropolitan area networks (MANs), and integrated service digital networks (ISDNs), or a wireless network such as wireless LANs, code division multiple access (CDMA), Bluetooth, and satellite communication, but the scope of the present disclosure is not limited thereto.

Referring to FIG. 1, the remote device 2 according to an embodiment of the present disclosure is illustrated as a single device, but is not limited thereto, and may include a plurality of devices capable of communicating with the medical liquid injection device 1000.

Referring to FIG. 1, the medical liquid injection device 1000 according to an embodiment of the present disclosure includes a housing 5 that forms an exterior and has a hollow interior, and an attachment part 6 that is disposed on one side (a lower side based on FIG. 1) of the housing 5 and is attached to the skin of a user.

A plurality of components may be disposed in an interior space of the housing 5 according to an embodiment of the present disclosure.

Referring to FIGS. 1 and 2, the medical liquid injection device 1000 according to an embodiment of the present disclosure may include the housing 5, the attachment part 6, a body part 10, a medical liquid discharge assembly 1100, and a reservoir 20, a needle assembly 30, and a battery 40.

The body part 10 is disposed inside the housing 5, and the reservoir 20, the needle assembly 30, the battery 40, and the medical liquid discharge assembly 1100 may be disposed on the body part 10.

The body part 10 may have conducting wires installed thereon to facilitate electrical connections to the driving part 1110 to be described later and the control module, for example, the body part 10 may be formed as a printed circuit board (PCB).

The reservoir 20 stores a medical liquid to be injected, and may be connected to the medical liquid discharge assembly 1100. Specifically, the reservoir 20 may be connected to the medical liquid discharge assembly 1100 through a first conduit P1.

Referring to FIG. 2, the reservoir 20 according to an embodiment of the present disclosure is disposed on the body part 10 disposed inside the housing 5, but is not limited thereto, and various modifications are possible, such as a configuration in which the reservoir 20 is installed on the outside of the housing 5 within the technical idea of supplying a medical liquid to the medical liquid discharge assembly 1100.

The reservoir 20 according to an embodiment of the present disclosure may include a plunger (not shown in the drawings), and the plunger may push the medical liquid in a preset direction while moving inside the reservoir 20.

The needle assembly 30 is disposed inside the housing 5, and may be disposed on one side of the body part 10. The needle assembly 30 is inserted into the skin of the user, so that the discharged medical liquid may be injected into the user.

The medical liquid discharge assembly 1100 may be installed between the needle assembly 30 and the reservoir 20 according to an embodiment of the present disclosure. Specifically, the needle assembly 30 may be connected to the reservoir 20 through a tube part 1140 and a second conduit P2, which are provided in the medical liquid discharge assembly 1100.

A medical liquid may be distributed in a preset amount or at a preset frequency from the medical liquid discharge assembly 1100 according to an embodiment of the present disclosure and discharged to the needle assembly 30 via the second conduit P2.

Referring to FIG. 2, the battery 40 according to an embodiment of the present disclosure may supply electricity to the medical liquid injection device 1000 to activate each component. While a single battery 40 is illustrated in the drawings, the battery 40 is not limited thereto, and may be set in various ways depending on the capacity, scope of use, duration of use, or the like of the medical liquid injection device 1000.

The battery 40 according to an embodiment of the present disclosure may be disposed adjacent to the driving part 1110 and may supply electricity to the driving part 1110. In addition, the battery 40 may be connected to the control module, and based on the electrical signals measured by a sensor unit, data about the number of rotations or rotational speed of the driving part 1110, the amount of medical liquid stored in the reservoir 20, the amount of medical liquid injected into the user, and the like may be measured.

Referring to FIGS. 2 to 4, 5A, and 6A, the medical liquid discharge assembly 1100 according to an embodiment of the present disclosure is disposed between the reservoir 20 and the needle assembly 30, and may be connected to the reservoir 20 via the first conduit P1 to receive a medical liquid from the reservoir 20.

The medical liquid discharge assembly 1100 is connected to the needle assembly 30 via the second conduit P2, and the medical liquid discharged from the medical liquid discharge assembly 1100 to the second conduit P2 may be introduced into the user's body via the needle assembly 30.

Referring to FIGS. 2 to 4, 5A, and 6A, the medical liquid discharge assembly 1100 according to an embodiment of the present disclosure is disposed inside the housing 5, and may be disposed on the body part 10.

The medical liquid discharge assembly 1100 according to an embodiment of the present disclosure may include the driving part 1110, the rotating part 1130, the tube part 1140, a pressure-applying part 1150, a locking part 1160, and a cover part 1170.

Referring to FIGS. 2, 3, and 4, the driving part 1110 according to an embodiment of the present disclosure is movable in a preset direction, and may generate a driving force and transmit the driving force to the rotating part 1130.

Although not shown in the drawings, the driving part 1110 according to an embodiment of the present disclosure may be electrically connected to a control module (not shown in the drawings), and can perform rotational and linear motions upon receiving electrical signals from the control module.

Although not shown in the drawings, the control module may control overall operations of the medical liquid injection device 1000. The control module is electrically in contact with and connected to internal devices such as the driving part 1110, the battery 40, and a plurality of sensor units, and may control operations thereof.

Based on data measured by an encoder unit (not shown in the drawings), the control module may calculate a rotation angle and a rotation speed of each of the driving part 1110 and the rotating part 1130, which receives a driving force from the driving part 1110 and rotates, and calculate a movement distance of the plunger, which is provided in the reservoir 20 and configured to discharge the medical liquid via the first conduit P1, and the amount of medical liquid discharged.

The control module can transmit and receive signals to and from the remote device 2, and the control module may receive signals from the external remote device 2 and transmit electrical signals to the driving part 1110.

The control module may drive the driving part 1110 by applying an electric signal to the driving part 1110, and the rotating part 1130 may rotate in one direction in response to the movement of the driving part 1110.

Referring to FIGS. 3 and 4, the driving part 1110 according to an embodiment of the present disclosure may include a wire part 1111, a lever part 1113, and a driving bar 1115.

The wire part 1111 according to an embodiment of the present disclosure deforms in shape upon receiving a current from the control module, and may be connected to the lever part 1113.

The wire part 1111 according to an embodiment of the present disclosure may contract or extend in length depending on a control signal applied from the control module.

In an optional embodiment, the wire part 1111 may be formed of a shape-memory alloy (SMA). The wire part 1111 may be formed of a known SMA material, and is not limited to a particular material. For example, the wire part 1111 may be formed of an alloy of nickel and titanium.

Referring to FIGS. 3 and 4, the wire part 1111 according to an embodiment of the present disclosure may be connected to the lever part 1113, and as the wire part 1111 extends or contracts upon receiving a current from the control module, the lever part 1113 may rotate in a clockwise or counterclockwise direction around a preset center axis of rotation.

Referring to FIGS. 3 and 4, as the lever part 1113 according to an embodiment of the present disclosure is rotated in the clockwise or counterclockwise direction due to the contraction or extension of the wire part 1111, the driving bar 1115 connected to the lever part 1113 is brought into contact with the rotating part 1130, specifically, with a gear part 1132 provided on a rotating plate 1131, and may transmit a driving force to the gear part 1132.

Referring to FIGS. 3 and 4, the driving bar 1115 according to an embodiment of the present disclosure may be formed in a straight shape, and may move in a preset direction (a vertical direction based on FIG. 4) in conjunction with the rotation of the lever part 1113 while being connected to the lever part 1113.

Referring to FIGS. 3 and 4, the driving bar 1115 according to an embodiment of the present disclosure can come into contact with the rotating part 1130, specifically, an outer peripheral surface of the gear part 1132 formed on one surface of the rotating plate 1131.

Accordingly, while reciprocating in a preset direction (vertical direction based on FIG. 4) in conjunction with the rotation of the lever part 1113, the driving bar 1115 may come into contact with and push the rotating part 1130, specifically the gear part 1132 formed on the rotating plate 1131.

Referring to FIG. 4, as the driving part 1110 transmits a driving force by coming into contact with and pushing the gear part 1132 formed on the rotating plate 1131, the rotating part 1130 may be rotated in a preset direction (in the counterclockwise direction based on FIG. 4).

Referring to FIG. 4, the driving bar 1115 according to an embodiment of the present disclosure may be provided in a plural number, and specifically, may be provided in pairs. The pair of driving bars 1115 may be disposed on both sides of the gear part 1132 formed on the rotating plate 1131, respectively.

The pair of driving bars 1115 may each be interlocked with the rotation of the lever part 1113 and move in opposite directions.

Referring to FIG. 4, when the wire part 1111 according to an embodiment of the present disclosure contracts upon receiving an electric signal from the control module, the lever part 1113 is interlocked with the contraction of the wire part 1111 and rotated in a first direction (the counterclockwise direction based on FIG. 4), and any one of the driving bars 1115 connected to the lever part 1113 may move from the lever part 1113 in a direction toward the rotating part 1130 (in an upward direction from the lower side based on FIG. 4).

Referring to FIG. 4, when the wire part 1111 according to an embodiment of the present disclosure extends upon receiving an electric signal from the control module, the lever part 1113 is interlocked with the extension of the wire part 1111 and rotated in a second direction (in the clockwise direction based on FIG. 4), and another driving bar 1115 facing the one driving bar 1115 with the gear part 1132 interposed therebetween may move in a direction toward the rotating part 1130 (in a downward direction from the upper side based on FIG. 4).

As the wire part 1111 contracts and extends upon receiving an electric signal from the control module, the lever part 1113 and the driving bar 1115 connected to the lever part 1113 may move in a preset direction to rotate the rotating part 1130 in a preset direction (the counterclockwise direction based on FIG. 4).

In an optional embodiment, the driving part 1110 may be driven by direct manipulation by the user, and various modifications are possible, such as a configuration in which the user externally applies a force to the driving part 1110 to perform a rotational motion and a linear motion.

Referring to FIGS. 3 and 4, the driving part 1110 according to an embodiment of the present disclosure may be disposed in the interior space of the housing 5 and disposed on the body part 10, and may transmit a driving force to the rotating part 1130.

The driving part 1110 according to an embodiment of the present disclosure is configured as a component forming the medical liquid discharge assembly 1100, but is not limited thereto, and various modifications are possible, such as a configuration in which the driving part 1110 and the medical liquid discharge assembly 1100 are disposed as separate components.

In an optional embodiment, the driving part 1110 may be any type of device that uses electricity to generate both suction and discharge forces for the medical liquid. For example, all types of pumps such as a mechanical displacement type micropump and an electromagnetic motion type micropump may be used.

The mechanical displacement type micropump is a pump that uses solid or fluid motion such as a gear or diaphragm to generate a pressure difference to induce fluid flow, and includes a diaphragm displacement pump, a fluid displacement pump, a rotary pump, and the like. The electromagnetic motion micropump is a pump that directly uses electrical or magnetic energy for fluid movement, and may include an electro-hydrodynamic pump (EHD), an electro-osmotic pump, a magneto-hydrodynamic pump, an electro-wetting pump, and the like.

Referring to FIGS. 3, 4, 5A to 5C, and 6A and 6B, the rotating part 1130 according to an embodiment of the present disclosure can come into contact with the driving part 1110, and may rotate in one direction in response to the movement of the driving part 1110.

Referring to FIGS. 3, 4, 5A, and 6A, the rotating part 1130 according to an embodiment of the present disclosure may include the rotating plate 1131 and a contact member 1133.

Referring to FIGS. 2 to 4, the rotating plate 1131 according to an embodiment of the present disclosure receives a driving force from the driving part 1110 and rotates, and may rotate in one direction around a preset center of rotation.

The center of rotation of the rotating part 1130 according to an embodiment of the present disclosure may be fixed in position to the body part 10.

Referring to FIG. 4, the gear part 1132 may be formed to protrude from one surface of the rotating plate 1131. The gear part 1132 may have a plurality of gear teeth formed along the circumference of the rotating plate 1131, centered around the center of rotation.

Referring to FIGS. 3 and 4, the gear part 1132 according to an embodiment of the present disclosure may be disposed to be in contact with the driving part 1110, specifically the driving bar 1115.

Referring to FIGS. 3 and 4, the rotating part 1130 according to an embodiment of the present disclosure may receive a driving force from the driving part 1110 and rotate in one direction (in the counterclockwise direction based on FIG. 4).

Specifically, as the wire part 1111, upon receiving the electric signal from the control module, contracts and extends, the lever part 1113 may rotate in the clockwise or counterclockwise direction around the preset center of rotation.

At this time, as the driving bar 1115 connected to the lever part 1113 moves linearly in a preset direction while in contact with the gear part 1132 formed on one surface of the rotating plate 1131, the gear part 1132 may be rotated.

Accordingly, the rotating plate 1131 may be rotated in one direction. The rotating plate 1131 may receive a driving force through the driving part 1110, specifically the driving bar 1115 and rotate in one direction (in the clockwise direction based on FIG. 5A).

Referring to FIGS. 5A to 5C, an engagement part 1135 may be formed to protrude from one surface of the rotating plate 1131 according to an embodiment of the present disclosure. The engagement part 1135 may be formed to protrude from another surface of the rotating plate 1131, which is opposite to one surface on which the gear part 1132 is formed, and can come into contact with the pressure-applying part 1150 to be described later.

Referring to FIGS. 5A to 5C, one end portion of the pressure-applying part 1150 may rest on the engagement part 1135, and as the rotating plate 1131, upon receiving a driving force from the driving part 1110, rotates in one direction (in the clockwise direction based on FIG. 5A), the engagement part 1135 may also be rotated integrally.

The engagement part 1135 may be formed to partially protrude in a preset section along the circumference of the rotating plate 1131, centered around the center of rotation. As a result, in an initial state, one end portion of the pressure-applying part 1150 may be in contact with the engagement part 1135 and may remain in the resting state.

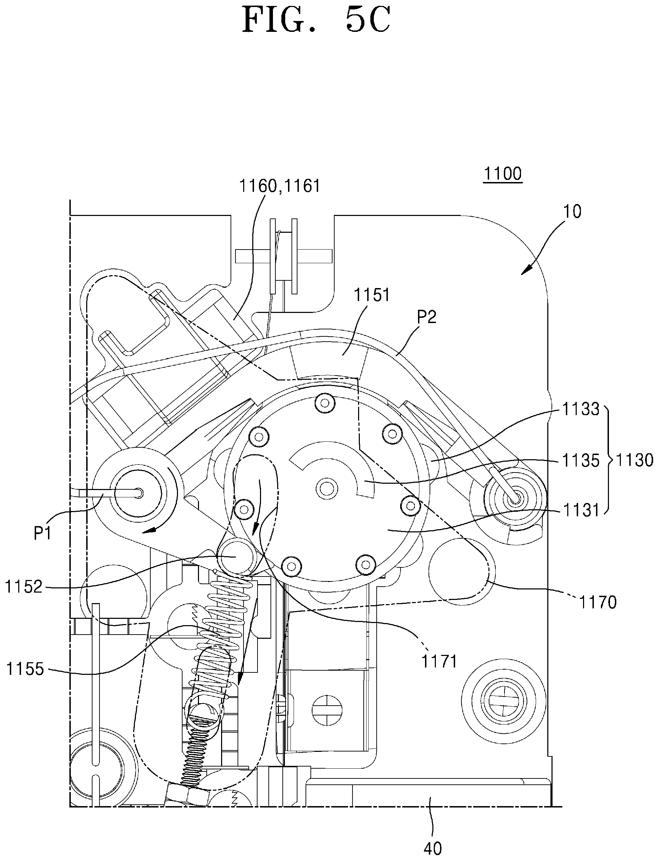

Referring to FIGS. 5A to 5C, as the rotating plate 1131 according to an embodiment of the present disclosure rotates, the engagement part 1135 formed in a preset section on the rotating plate 1131 also rotates, and at a moment when the one end portion of the pressure-applying part 1150 in contact with the engagement part 1135 deviates from the engagement part 1135, the pressure-applying part 1150 rotates around the preset center of rotation in a direction (in the clockwise direction based on FIG. 5C) surrounding the rotating part 1130.

The pressure-applying part 1150 may surround the rotating part 1130 while rotating in one direction (clockwise direction based on FIG. 5C), and the tube part 1140 disposed between the pressure-applying part 1150 and the rotating part 1130 may be compressed by the contact member 1133 provided in the rotating part 1130.

As the pressure-applying part 1150 surrounds the rotating part 1130, and the tube part 1140 disposed between the pressure-applying part 1150 and the rotating part 1130 is compressed, a cross-sectional area of the tube part 1140 may become zero, and the medical liquid that is accommodated inside the tube part 1140 through the compressed section may be prevented from leaking out to a region outside the compressed section.

Referring to FIGS. 4 and 5A, the contact member 1133 according to an embodiment of the present disclosure is rotatably installed on the rotating plate 1131, and may be connected to the rotating plate 1131 to be in contact with the tube part 1140.

Referring to FIGS. 2, 4, and 5A, the contact member 1133 according to an embodiment of the present disclosure may have an outer peripheral surface formed as a curved surface. Specifically, the contact member 1133 is formed in a cylindrical shape, and may be rotatably disposed on the rotating plate 1131.

The center of rotation of the contact member 1133 may be located on the rotating plate 1131, and the outer peripheral surface of the contact member 1133 may be disposed outward from the outer peripheral surface of the rotating plate 1131 with respect to the center of rotation of the rotating plate 1131.

As a result, the contact member 1133 may come into contact with the tube part 1140, which is disposed between the rotating part 1130 and the pressure-applying part 1150, and may compress the contact member 1133 as the pressure-applying part 1150 rotates toward the rotating part 1130.

Referring to FIGS. 2, 4, and 5A, a plurality of contact members 1133, according to an embodiment of the present disclosure, may be provided, and the plurality of contact members 1133 may be disposed to be spaced apart at certain intervals along the circumference of the rotating plate 1131 with respect to the center.

A distance from the center of rotation of the rotating plate 1131 to the center of rotation of the plurality of contact members 1133 may be formed equally.

Referring to FIGS. 2 and 5A, the contact member 1133 according to an embodiment of the present disclosure may be rotatably connected to the rotating plate 1131, and specifically, may be connected to the rotating plate 1131 through a pin part (not shown in the drawings).

A longitudinal central axis of the pin part may be the same as a longitudinal central axis of the contact member 1133, and the contact member 1133 may rotate on the rotating plate 1131 with the pin part as the center axis of rotation thereof.

Referring to FIG. 5C, as the pressure-applying part 1150 according to an embodiment of the present disclosure rotates in a direction approaching the rotating part 1130, and the tube part 1140 disposed between the pressure-applying part 1150 and the rotating part 1130 may be compressed by the pressure-applying part 1150 and the rotating part 1130.

Referring to FIG. 5C, the tube part 1140 may be compressed at a plurality of points by the plurality of contact members 1133, and the cross-sectional area of the tube part 1140 may be zero at the plurality of points.

Specifically, the tube part 1140 may be compressed at two points by the pressure-applying part 1150 and a pair of contact members 1133, causing the cross-sectional area thereof to become zero, and accordingly, a set amount of medical liquid may be set based on an internal volume of the tube part 1140 between these two points.

As the driving part 1110 according to an embodiment of the present disclosure is driven upon receiving an electric signal from the control module, the driving part 1110 may transmit a driving force to the rotating part 1130 and the rotating part 1130 may rotate in one direction (clockwise direction based on FIG. 5C).

As the rotating plate 1131 rotates, the contact member 1133 rotatably connected to the rotating plate 1131 also rotates, causing the plurality of points compressed by the pressure-applying part 1150 and the contact member 1133 to move.

The set amount of medical liquid, which is accommodated between the plurality of points, may move inside the tube part 1140 and may flow into the needle assembly 30 via the second conduit P2. Accordingly, the set amount of medical liquid can be introduced into the user's body via the needle assembly 30.

Referring to FIG. 5C, as the pressure-applying part 1150 surrounds the rotating part 1130, the tube part 1140 is compressed at a plurality of points by the plurality of contact members 1133 provided on the rotating part 1130, and the rotating part 1130 receives a driving force from the driving part 1110 and rotates, the set amount of medical liquid accommodated between the plurality of points, at which the tube part 1140 is compressed, may move and flow into the second conduit P2.

In an optional embodiment, the amount of medical liquid accommodated between a pair of points may be set differently by varying the number of contact members 1133. For example, when the number of contact members 1133 is increased, the amount of the distributed medical liquid may be set to be relatively small, and the medical liquid finally discharged may be injected in a set amount.

Referring to FIGS. 2, 4, 5A, and 6A, the tube part 1140 according to an embodiment of the present disclosure may be formed of a flexible material and may be disposed between the rotating part 1130 and the pressure-applying part 1150 to be described later.

The tube part 1140 according to an embodiment of the present disclosure may be formed with a hollow interior, through which the medical liquid may flow, and may be disposed adjacent to the rotating part 1130.

Referring to FIGS. 2, 4, 5A, and 6A, the tube part 1140 according to an embodiment of the present disclosure can come into contact with the pressure-applying part 1150 and the rotating part 1130 from both sides, and the tube part 1140 may be compressed by the pressure-applying part 1150 and the rotating part 1130.

Referring to FIGS. 2 and 4, the tube part 1140 according to an embodiment of the present disclosure may have both end portions fixed in position to the pressure-applying part 1150, specifically, a pressure-applying body 1151. One end portion of the tube part 1140 may be connected to the first conduit P1, and may receive a medical liquid from the reservoir 20 via the first conduit P1.

Another end portion of the tube part 1140, which is opposite to one end portion connected to the first conduit P1, may be connected to the second conduit P2. The tube part 1140 may supply a medical liquid to the needle assembly 30 via the second conduit P2, and the medical liquid may be discharged to the user via the needle assembly 30.

As the pressure-applying part 1150 moves toward the rotating part 1130 with both end portions of the tube part 1140 fixed in position to the pressure-applying part 1150, the tube part 1140 may move, in conjunction with the pressure-applying part 1150, toward the rotating part 1130.

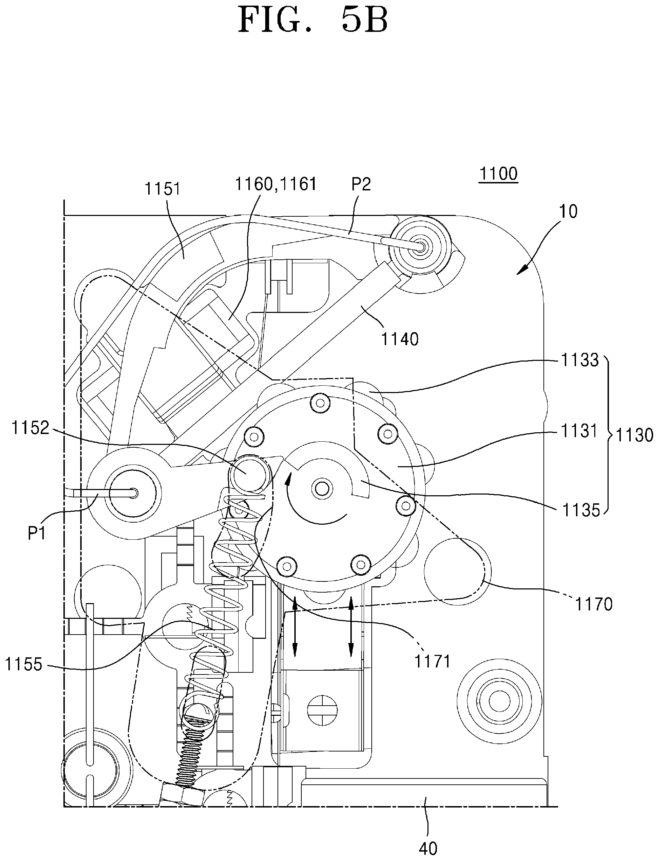

Referring to FIGS. 5B and 5C, as the pressure-applying part 1150 moves toward the rotating part 1130, the tube part 1140 connected to the pressure-applying part 1150 approaches the rotating part 1130, specifically the contact member 1133, and as the pressure-applying part 1150 moves further while the tube part 1140 is in contact with the contact member 1133, the tube part 1140 may be compressed between the pressure-applying part 1150 and the rotating part 1130.

Referring to FIG. 5C, the tube part 1140 according to an embodiment of the present disclosure may be compressed at a plurality of points. Specifically, the tube part 1140 may be in contact with and compressed at a pair of points by a pair of contact members 1133, and an internal cross-sectional area of the tube part 1140 may become zero at the pair of points.

Since the internal cross-sectional area of the tube part 1140 at the pair of points becomes zero, a preset amount of medical liquid may be accommodated in the interior space of the tube part 1140 between the pair of points, and as the rotating part 1130 receives a driving force from the driving part 1110 and rotates, the medical liquid may flow within the tube part 1140 as the plurality of contact members 1133 push the tube part 1140.

As the rotating part 1130 rotates, the plurality of contact members 1133, which are rotatably connected to the rotating plate 1131, may cause the medical liquid accommodated inside the tube part 1140 to flow into the needle assembly 30 by a preset amount via the second conduit P2.

Referring to FIGS. 5C and 6B, since the tube part 1140 according to an embodiment of the present disclosure includes a flexible material and can be deformed in shape, a curved section with a preset radius of curvature may be formed in the tube part 1140 as the pressure-applying part 1150 moves in a direction toward the rotating part 1130 and comes into contact with the tube part 1140 and the rotating part 1130, specifically, the contact member 1133.

Referring to FIGS. 2 and 4, a curved surface part (not shown in the drawings) may be formed on one surface of the pressure-applying part 1150, specifically the pressure-applying body 1151, which faces the tube part 1140, to correspond to a curved section formed when the tube part 1140 is in contact with the contact member 1133 and compressed.

In addition, since the tube part 1140 includes a flexible material, the tube part 1140 can be in contact with, compressed by, and deformed in shape by the rotating part 1130, specifically the contact member 1133 and the pressure-applying part 1150, and the internal cross-sectional area of the tube part 1140 may become zero at a pair of points.

In addition, since the internal cross-sectional area of the tube part 1140 becomes zero at a pair of points, a set amount of medical liquid accommodated between the pair of points may flow within the tube part 1140 while maintaining a set amount, and the set amount of medical liquid may be discharged to the needle assembly 30 via the second conduit P2.

Referring to FIGS. 2, 4, 5A, and 6A, the pressure-applying part 1150 according to an embodiment of the present disclosure is disposed facing the rotating part 1130 with the tube part 1140 interposed therebetween, and can come into contact with the rotating part 1130.

Referring to FIGS. 5A to 5C, the pressure-applying part 1150 according to an embodiment of the present disclosure may apply pressure to the tube part 1140 while coming into contact therewith, in response to the rotation of the rotating part 1130.

The pressure-applying part 1150 according to an embodiment of the present disclosure may apply pressure to the tube part 1140 so that the tube part 1140 is compressed at a contact point where the pressure-applying part 1150 comes into contact with the tube part 1140.

Referring to FIGS. 2, 4, 5A, and 6A, the pressure-applying part 1150 according to an embodiment of the present disclosure may include the pressure-applying body 1151 and a pulling member 1155.

The pressure-applying body 1151 according to an embodiment of the present disclosure may be disposed on the body part 10 and may rotate in a preset direction (clockwise direction based on FIG. 5C) around a preset center axis of rotation.

Both end portions of the tube part 1140 may be connected to the pressure-applying body 1151 according to an embodiment of the present disclosure and fixed in position.

Referring to FIG. 4, a curved portion having a preset curvature may be formed on one surface of the pressure-applying part 1150, specifically the pressure-applying body 1151, facing the tube part 1140.

As a result, when the pressure-applying part 1150 moves toward the rotating part 1130, and the tube part 1140 disposed between the pressure-applying part 1150 and the rotating part 1130 is deformed to have a certain curvature, the pressure-applying part 1150 may support the tube part 1140 on a side of the contact member 1133, which is opposite the side that compress the tube part 1140, so that the tube part 1140 may remain in the compressed state.

Referring to FIGS. 5A to 5C, one end portion of the pressure-applying body 1151 according to an embodiment of the present disclosure may rest on and be in contact with the rotating part 1130, specifically the engagement part 1135, which is formed to protrude from the rotating plate 1131.

Referring to FIG. 5A, one end portion of the pressure-applying body 1151 is in a state of being in contact with the engagement part 1135, and referring to FIG. 5B, the rotating part 1130 receives a driving force from the driving part 1110 and rotates in one direction (clockwise direction based on FIG. 5B) around the center of the rotating part 1130, and the engagement part 1135, which is formed to protrude from the rotating part 1130, specifically, the rotating plate 1131, also rotates around the center of the rotating part 1130.

The engagement part 1135 is formed in a certain section of the rotating part 1130 in a circumferential direction with respect to the center, of the rotating part, and as the rotating part 1130 rotates, the contact between the pressure-applying body 1151 and the engagement part 1135 may be released.

Referring to FIG. 5C, when the contact between the pressure-applying body 1151 and the engagement part 1135, which is formed to protrude from the rotating plate 1131, is released, the pressure-applying body 1151 is rotated in one direction (clockwise direction based on FIG. 5C) around the center axis of rotation by the pulling member 1155 to be described later, and is rotated in a direction approaching the rotating part 1130.

Referring to FIGS. 5A to 5C, a protrusion 1152 may be formed to protrude from the pressure-applying body 1151 according to an embodiment of the present disclosure. The protrusion 1152 may be formed to extend along a central axis of the pressure-applying body 1151 in the longitudinal direction, which is formed parallel to the center axis of rotation of the pressure-applying body 1151.

The protrusion 1152, which is formed on the pressure-applying body 1151, may move along a guide part 1171, which is formed in the shape of a hole or groove on the cover part 1170 and will be described later. As the protrusion 1152 moves along the guide part 1171 formed on the cover part 1170, the pressure-applying body 1151 may be stably rotated along a preset rotation path.

As the rotational movement path of the pressure-applying body 1151 is stably provided, the pressure-applying part 1150 may move close to the rotating part 1130 so as to surround the rotating part 1130, and the pressure-applying body 1151 may compress, in conjunction with the rotating part 1130, the tube part 1140.

The pressure-applying part 1150, specifically the pressure-applying body 1151, may compress, along which the plurality of contact members 1133 provided on the rotating part 1130, the tube part 1140 from both sides. As a result, the internal cross-sectional area of the tube part 1140, which is compressed by the plurality of contact members 1133, specifically, a pair of contact members 1133 and the pressure-applying part 1150, may become zero, and a set amount of medical liquid may be accommodated between a pair of points of the tube part 1140 compressed by the pair of contact members 1133.

In addition, as the rotating part 1130 receives a driving force from the driving part 1110 and rotates, a set amount of medical liquid preset by another pair of contact members 1133 may sequentially flow within the tube part 1140 and may flow to the needle assembly 30 via the second conduit P2.

Referring to FIGS. 2, 5A to 5C, 6A, and 6B, the pulling member 1155 according to an embodiment of the present disclosure is connected to the pressure-applying body 1151, and may pull the pressure-applying body 1151 in a preset direction.

The pulling member 1155 according to an embodiment of the present disclosure may be formed of an elastic material and may exhibit an elastic restoring force in a preset direction.

One side of the pulling member 1155 may be fixed in position to the body part 10, and another side of the pulling member 1155 opposite to the one side may be connected to the pressure-applying body 1151. Another side of the pulling member 1155, which is connected to the pressure-applying body 1151, may exhibit an elastic restoring force in a direction of one side of the pulling member 1155 (in the downward direction from the upper side based on FIG. 5C), which is fixed in position to the body part 10.

Referring to FIGS. 5A to 5C, the pulling member 1155 may remain in the extended state while one end portion of the pressure-applying body 1151 is in contact with the rotating part 1130, specifically, the engagement part 1135 formed to protrude from the rotating plate 1131.

In contrast, when the pressure-applying body 1151 and the engagement part 1135 are released from contact with each other and spaced apart from each other as the rotating part 1130 receives a driving force from the driving part 1110 and rotates in a preset direction (clockwise direction based on FIG. 5B), the pulling member 1155 is moved in a direction of one side of the pulling member 1155, which is fixed in position to the body part 10, by the elastic restoring force.

As a result, the pressure-applying body 1151 connected to the pulling member 1155 moves together, and specifically, the pressure-applying body 1151 moves in the clockwise direction (based on FIG. 5c) with respect to the center axis of rotation.

The pulling member 1155 according to an embodiment of the present disclosure is formed as a coil-shaped spring, but is not limited thereto, and various modifications are possible within the technical idea of exhibiting an elastic restoring force in a preset direction, specifically in the direction in which the pressure-applying part 1150 approaches the rotating part 1130.

In the present disclosure, the pulling member 1155 is fixed in position to the body part 10 and is provided as a single component, but the present disclosure is not limited thereto, and various modifications are possible, such as a configuration in which a plurality of pulling members 1155 are connected to the pressure-applying body 1151 and exhibit an elastic restoring force a direction in which the pressure-applying part 1150 approaches the rotating part 1130.

Referring to FIGS. 2, 3, 5A to 5C, 6A, and 6B, the locking part 1160 according to an embodiment of the present disclosure is movable in a preset direction, and while the pressure-applying part 1150 is applying pressure to the tube part 1140, the locking part 1160 may come into contact with and support one surface of the pressure-applying part 1150 and fix the position of the pressure-applying part 1150.

The locking part 1160 according to an embodiment of the present disclosure may include a locking body 1161 and a pushing part 1165.

Referring to FIGS. 2, 3, 5A to 5C, 6A, and 6B, the locking body 1161 can come into contact with the pressure-applying part 1150, and may be disposed on the body part 10. A step part 1162, which is formed by varying the height of the locking body 1161, may be formed on the locking body 1161.

Referring to FIGS. 6A and 6B, the locking body 1161 according to an embodiment of the present disclosure can come into contact with the pushing part 1165, and move in a preset direction (in the upward direction from the lower side based on FIG. 6B) by the elastic restoring force of the pushing part 1165.

One surface (upper surface based on FIG. 6A) of the locking body 1161 according to an embodiment of the present disclosure can come into contact with the pressure-applying part 1150, specifically, one surface (lower surface based on FIG. 6A) of the pressure-applying body 1151.

Referring to FIG. 6A, the state is illustrated before the pressure-applying part 1150 rotates in a direction approaching the rotating part 1130, and in this state, a first region A1, which is formed at a relatively high height on the locking body 1161, is in contact with the pressure-applying body 1151 and the pushing part 1165 may be in a compressed state.

Referring to FIG. 6B, in FIG. 5C, the pressure-applying part 1150, specifically the pressure-applying body 1151, moves in a direction approaching the rotating part 1130, that is, in the direction of compressing and applying pressure to the tube part 1140, and accordingly, the contact between the first region A1 of the locking body 1161, which is formed at a relatively high height, and the pressure-applying body 1151 is moved in a direction in which the locking body 1161 is spaced apart from the body part 10 (in the upward direction from the lower side based on FIG. 6B) by the elastic restoring force of the pushing part 1165.

Referring to FIGS. 6A and 6B, the step part 1162 may be formed in the locking body 1161, and due to the step part 1162, the locking body 1161 may be divided into the first region A1 formed at a relatively higher height and a second region A2 formed at a relatively lower height than the first region A1.

Referring to FIG. 6B, since the locking body 1161 is moved upward by the pushing part 1165, and the step part 1162 is formed in the locking body 1161 due to the height difference between the first region A1 and the second region A2, the first region A1 of the locking body 1161 may be disposed on a movement path of the pressure-applying body 1151.

Referring to FIG. 6B, as the step part 1162 formed in the locking body 1161 is disposed on a rotational path of the pressure-applying body 1151 while coming into contact with and supporting the pressure-applying body 1151, the elastic restoring force of the tube part 1140, which includes a flexible material, may prevent the pressure-applying body 1151 from being pushed in such a direction that the pressure-applying body 1151 is spaced apart from the rotating part 1130.

That is, the state in which the tube part 1140 is compressed by the pressure-applying part 1150 and the rotating part 1130, resulting in a zero internal cross-sectional area, may be maintained, and the leakage of medical liquid toward the space between the compressed points of the tube part 1140, which are compressed by the rotating part 1130, specifically the plurality of contact members 1133 and the pressure-applying body 1151.

In addition, a set amount of medical liquid accommodated between the plurality of points, in which the internal cross-sectional area of the tube part 1140 is zero, flows within the tube part 1140 by the rotation of the rotating part 1130, and flows to the needle assembly 30 via the second conduit P2.

Referring to FIGS. 6A and 6B, the body part 10 according to an embodiment of the present disclosure may have a side wall 11 formed to protrude in an outward direction, and the side wall 11 may be formed to extend in a direction parallel to a moving direction (in the vertical direction based on FIG. 6B) of the locking body 1161.

As a result, a movement path of the locking body 1161 may be stably provided, and the state in which the locking body 1161 restricts the movement of the pressure-applying body 1151 may be maintained.

Referring to FIGS. 2, 5A, and 6A, the cover part 1170 according to an embodiment of the present disclosure is disposed outside the pressure-applying part 1150, and may cover the pressure-applying part 1150. The cover part 1170 may be disposed on the body part 10 and may be fixed in position to the body part 10 at least one or more points.

Referring to FIGS. 5A to 5C, the guide part 1171 may be formed in the shape of a groove or hole on the cover part 1170, and the protrusion 1152 may be disposed on an inner side of the guide part 1171 that is formed to protrude from the pressure-applying part 1150, specifically the pressure-applying body 1151.

As a result, when the pressure-applying body is rotationally moved in a direction of approaching the rotating part 1130 due to the elastic restoring force of the pulling member 1155, the protrusion 1152 formed to protrude from the pressure-applying body 1151 may move along the guide part 1171 formed on the cover part 1170, thereby providing a stable rotational movement path for the pressure-applying body 1151.

In addition, as the pressure-applying body 1151 moves along a preset rotational movement path, the pressure-applying body 1151 may, in conjunction with the rotating part 1130, compress and apply pressure to the tube part 1140.

Hereinafter, the configuration, operating principle, and effects of a medical liquid injection device according to another embodiment of the present disclosure will be described.

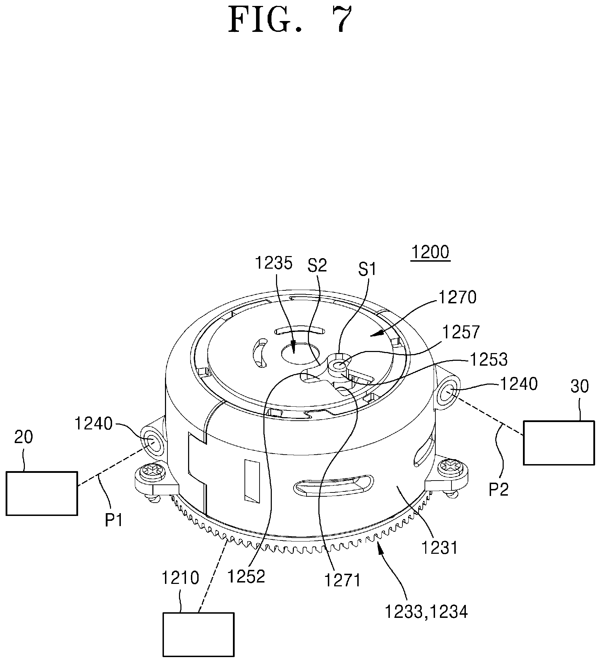

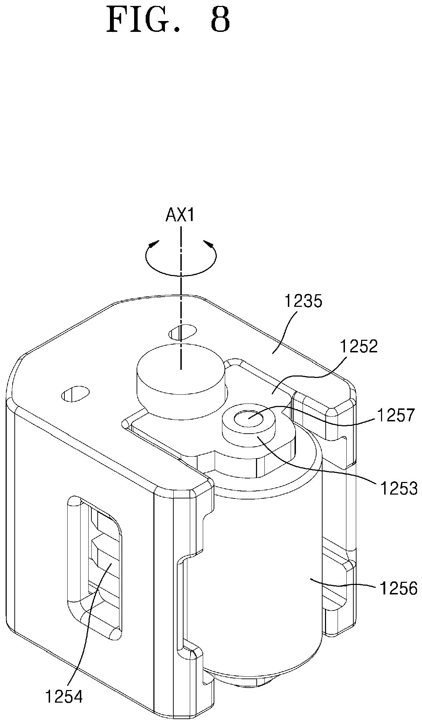

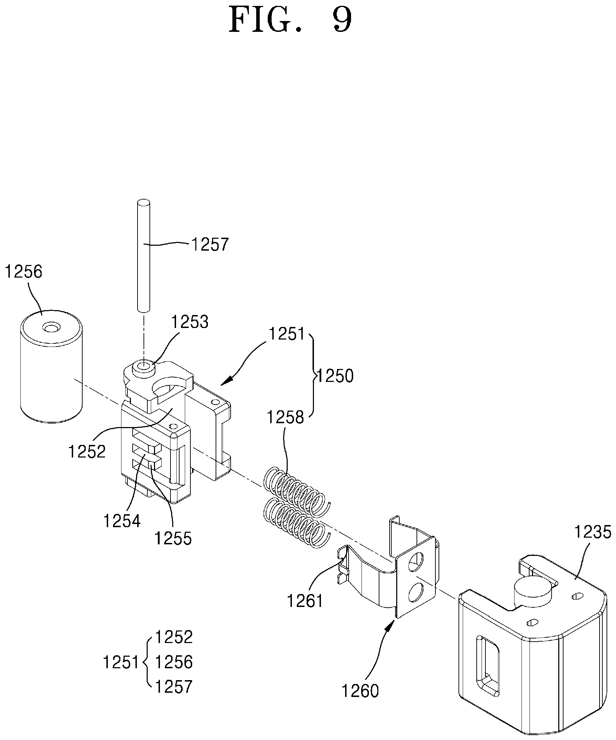

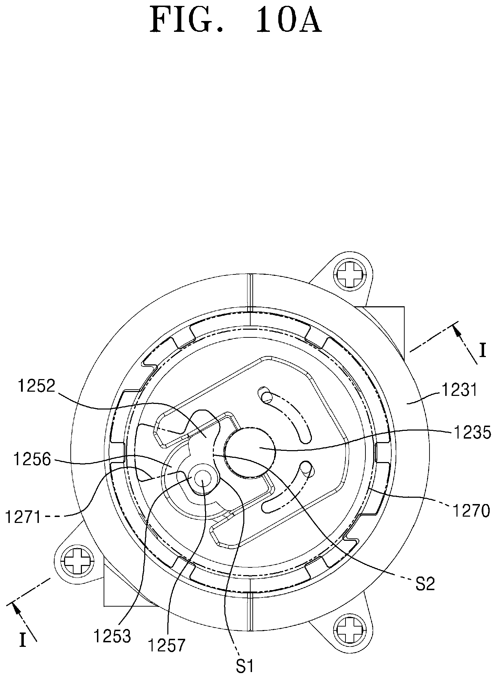

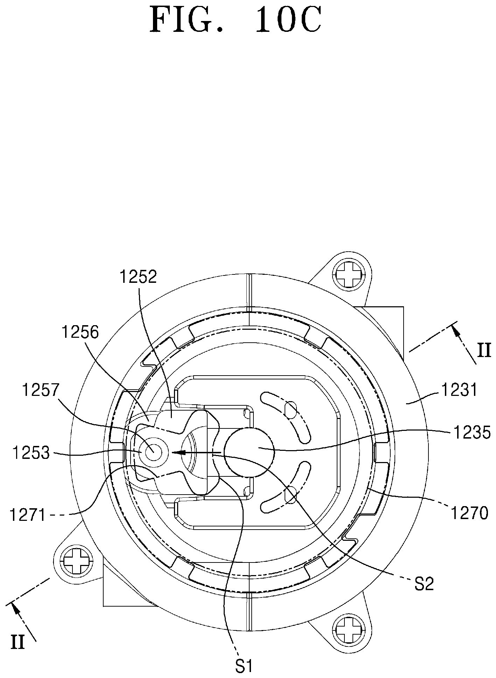

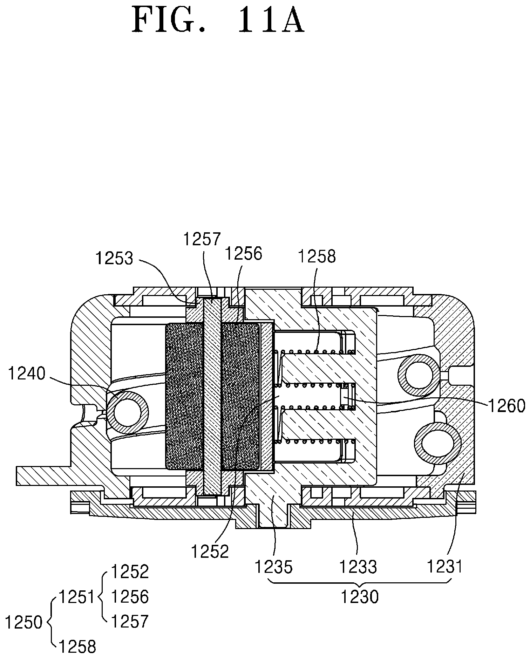

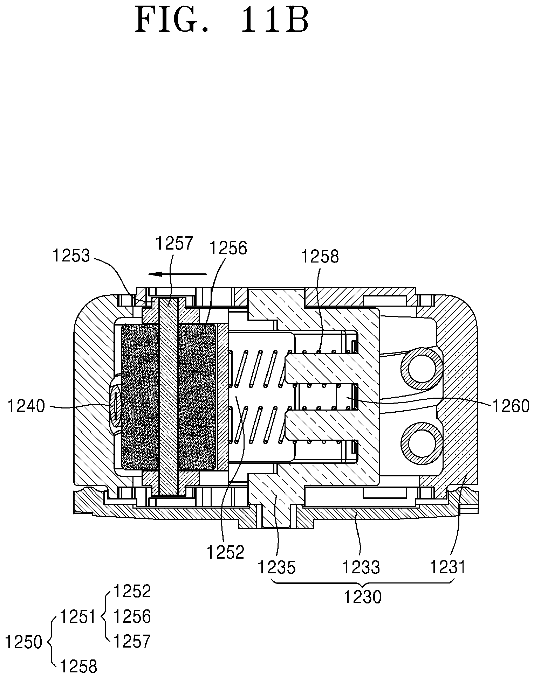

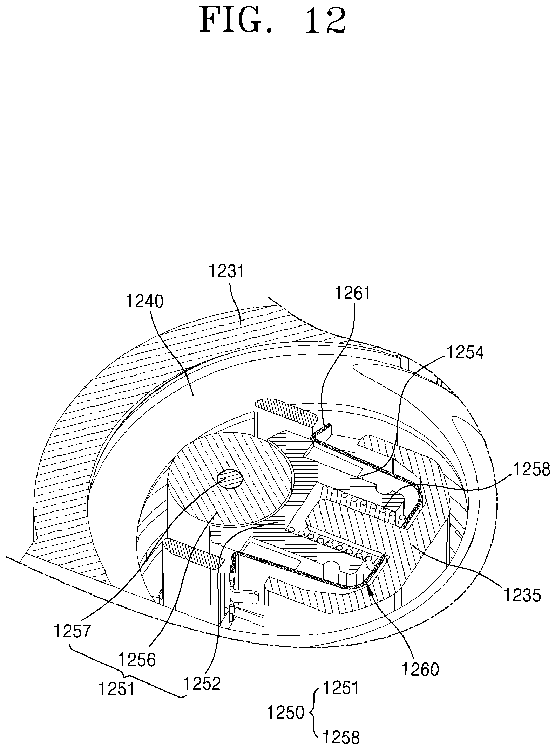

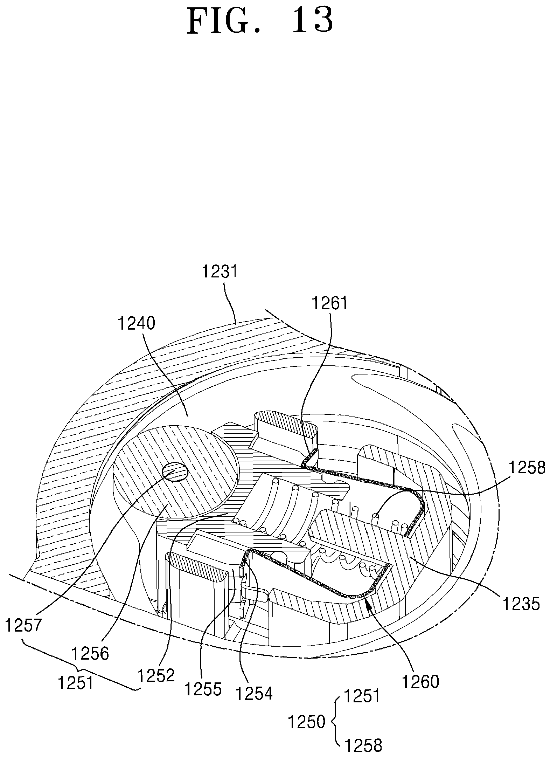

FIG. 7 is a perspective view illustrating a medical liquid discharge assembly according to another embodiment of the present disclosure. FIG. 8 is a perspective view illustrating a rotating part and a pressure-applying part according to another embodiment of the present disclosure. FIG. 9 is an exploded perspective view illustrating the rotating part and the pressure-applying part according to another embodiment of the present disclosure. FIGS. 10A to 10C are views illustrating an operation process of the medical liquid discharge assembly according to another embodiment of the present disclosure. FIG. 11A is a cross-sectional view taken along line I-I of FIG. 10A. FIG. 11B is a cross-sectional view taken along line II-II of FIG. 10C. FIG. 12 is a view partially illustrating the state of FIG. 10A. FIG. 13 is a view partially illustrating the state of FIG. 10C.

Similar to the medical liquid injection device 1000 according to an embodiment of the present disclosure illustrated in FIG. 1, the medical liquid injection device according to another embodiment of the present disclosure may include a housing 5, an attachment part 6, and a body part 10, a reservoir 20, a needle assembly 30, a battery 40, a medical liquid discharge assembly 1200, and a control module (not shown in the drawings).

The medical liquid injection device according to another embodiment of the present disclosure, except for the configuration of the medical liquid discharge assembly 1200, has the same configuration, operating principle, and effects of the body part 10, the reservoir 20, the needle assembly 30, the battery 40, and the control module as those of the medical liquid injection device 1000 according to an embodiment of the present disclosure, and thus, detailed descriptions of these components are omitted to the extent of overlapping.

Hereinafter, the description will focus on the configuration, operating principle, and effects of the medical liquid discharge assembly 1200 provided in the medical liquid injection device according to another embodiment of the present disclosure.

The medical liquid discharge assembly 1200 according to another embodiment of the present disclosure is disposed between the reservoir 20 and the needle assembly 30, and may be connected to the reservoir 20 via the first conduit P1, and may receive a medical liquid from the reservoir 20.

The medical liquid discharge assembly 1200 is connected to the needle assembly 30 via the second conduit P2, and the medical liquid discharged from the medical liquid discharge assembly 1200 to the second conduit may be introduced into the user's body via the needle assembly.

Referring to FIG. 7, the medical liquid discharge assembly 1200 according to another embodiment of the present disclosure is illustrated, and may be disposed inside the housing and disposed on the body part.

Referring to FIGS. 7 to 9, 10A, 11A, and 12, a driving part 1210 according to another embodiment of the present disclosure moves in a preset direction, and may transmit a driving force to a rotating part 1230.

The driving part 1210 can come into contact with the rotating part 1230, and the rotating part 1230 may rotate in one direction in response to the movement of the driving part 1210.

The driving part 1210 according to another embodiment of the present disclosure may receive an electric signal from the control module and move in a preset direction. In an optional embodiment, the driving part 1210 is capable of linear or rotational movement.

Referring to FIG. 7, according to another embodiment of the present disclosure, a gear part 1234 may be formed along an outer peripheral surface of the rotating part 1230, specifically a rotating plate 1233, and the driving part 1210 may come into contact with the gear part 1234 formed on the rotating plate 1233 and transmit a driving force to the rotating plate 1233.

The driving part 1210 according to another embodiment of the present disclosure may be configured in the same manner as the driving part 1110 according to an embodiment of the present disclosure. However, the driving part 1210 is not limited thereto, and various modifications are possible within the technical idea of receiving a driving force from the driving part 1210 to rotate the rotating part 1230 in a preset direction.

Referring to FIGS. 7, 10A to 10C, 11A, and 12, the rotating part 1230 according to another embodiment of the present disclosure may include a unit housing part 1231, a rotating plate 1233, and a connection part 1235.

Referring to FIG. 7, the unit housing part 1231 is installed on the body part, and may be fixed in position to the body part at least one or more points. In the present disclosure, the unit housing part 1231 is fixed in position to the body part by coupling members (a reference numeral is not assigned) at three points, but is not limited thereto, and various modifications are possible, such as a configuration in which the unit housing part 1231 is fixed in position at four or more points within the technical idea of maintaining the center of the unit housing part 1231.

The connection part 1235, a tube part 1240, a pressure-applying part 1250, and a locking part 1260, which will be described later, may be installed inside the unit housing part 1231 according to another embodiment of the present disclosure, and a cover part 1270 may cover the unit housing part 1231 and may be connected to the unit housing part 1231.

Referring to FIGS. 7 and 10A to 10C, the cover part 1270, on which a guide part 1271 to be described later is formed, may be fixed in position to the unit housing part 1231, and accordingly, a protrusions 1253, which will be described later, may be interlocked with the rotation of the rotating plate 1233 and is movable on the guide part 1271.

Referring to FIGS. 7 and 11A, the tube part 1240 may be installed in the unit housing part 1231, and both end portions of the tube part 1240 may be connected to the first conduit P1 and the second conduit P2, respectively. The first conduit may be connected to the reservoir 20, a medical liquid may flow into the tube part 1240 from the reservoir 20 via the first conduit P1, and the medical liquid flowing within the tube part 1240 may flow to the needle assembly 30 via the second conduit P2 connected to another end portion of the tube part 1240, which is opposite to one end portion connected to the first conduit P1.

Referring to FIGS. 12 and 13, an inner surface of the unit housing part 1231 may be formed as a curved surface to correspond to the shape of the tube part 1240, which has a certain curvature and in which a curved section is formed.

Accordingly, the pressure-applying part 1250, which will be described later, can compress and apply pressure to the tube part 1240 from one side, and the unit housing part 1231 facing the pressure-applying part 1250 with the tube part 1240 interposed therebetween comes into contact with and supports the tube part 1240 so that the tube part 1240 remains in the compressed state.

Referring to FIGS. 7, 11A, and 11B, the rotating plate 1233 according to another embodiment of the present disclosure receives a driving force from the driving part 1210, and may rotate in one direction in response to the movement of the driving part 1210.

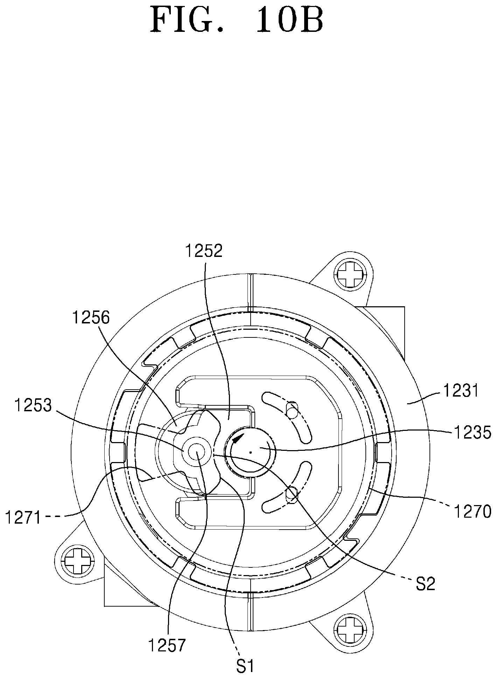

The gear part 1234 may be formed around the outer peripheral surface of the rotating plate 1233, and the driving part 1210 may come into contact with the gear part 1234. When the driving part 1210 receives an electric signal from the control module, the driving part 1210 can rotate in a preset direction (clockwise direction based on FIG. 10B).

As the rotating plate 1233 rotates, the connection part 1235 connected to the rotating plate 1233 may rotate in a preset direction in which the connection part 1235 can rotate in conjunction with the rotation of the rotating plate 1233,

Referring to FIG. 8, the connection part 1235 may be formed to extend in a direction of a longitudinal central axis AX1 and may rotate in a preset direction with the longitudinal central axis AX1 as the center axis of rotation.

Referring to FIG. 8, the longitudinal central axis AX1, i.e., the center axis of rotation of the connection part 1235 according to another embodiment of the present disclosure may share the center axis of rotation with the rotating plate 1233. Accordingly, when the rotating plate 1233 receives a driving force from the driving part 1210 and rotates, the connection part 1235 and the pressure-applying part 1250, which is installed on the connection part 1235, may be rotated together.

Referring to FIGS. 7, 11A, 11B, 12, and 13, the tube part 1240 according to another embodiment of the present disclosure includes a flexible material, and may be disposed adjacent to the rotating part 1230.

Specifically, the tube part 1240 may be disposed along an inner surface perimeter of the unit housing part 1231. The tube part 1240 may be disposed between the inner surface of the unit housing part 1231 and the pressure-applying part 1250. The tube part 1240 may be formed with a hollow interior, through which a medical liquid may flow, and may be disposed adjacent to the rotating part 1230.

Referring to FIGS. 11B and 13, the inner surface of the unit housing part 1231 and the pressure-applying part 1250, specifically a pressure-applying member 1256 can come into contact with the tube part 1240 according to another embodiment of the present disclosure from both sides, and the tube part 1240 may be compressed by the pressure-applying part 1250 and the rotating part 1230, specifically the unit housing part 1231.

Referring to FIG. 7, both end portions of the tube part 1240 according to another embodiment of the present disclosure may be fixed in position to the rotating part 1230, specifically, the unit housing part 1231. One end portion of the tube part 1240 may be connected to the first conduit P1, and may receive a medical liquid from the reservoir 20 via the first conduit P1.

Another end portion of the tube part 1240, which is opposite to one end portion connected to the first conduit P1, may be connected to the second conduit P2. The tube part 1240 may supply a medical liquid to the needle assembly 30 via the second conduit P2, and the medical liquid may be discharged to the user through the needle assembly 30.

Referring to FIGS. 11B and 13, the tube part 1240 according to another embodiment of the present disclosure may be compressed at least one point. Specifically, the tube part 1240 may be in contact with and compressed by the pressure-applying member 1256 at least one point, and an internal cross-sectional area of the tube part 1240 may become zero at the point.

Due to the internal cross-sectional area of the tube part 1240 becoming zero at a preset point, as the rotating part 1230 receives a driving force from the driving part 1210 and rotates, residual air in the tube part 1240 can be reliably removed during a priming operation in which the residual air in the tube part 1240 is stably discharged via the needle assembly.

In addition, due to the internal cross-sectional area of the tube part 1240 becoming zero at a preset point, the rotating part 1230, specifically the rotating plate 1233, may rotate in conjunction with the rotation of the connection part 1235 while the pressure-applying member 1256 compresses and applies pressure to the tube part 1240, and a medical liquid in an amount corresponding to the amount of rotation of the pressure-applying part 1250 may flow within the tube part 1240 and flow into the needle assembly 30 via the second conduit P2.

Referring to FIGS. 11B and 13, since the tube part 1240 includes a flexible material, the tube part 1240 may be in contact with, compressed by, and deformed in shape by the pressure-applying part 1250, specifically the pressure-applying member 1256, and may have a zero internal cross-sectional area at least one point.

In addition, since the internal cross-sectional area of the tube part 1240 becomes zero at least one point, a set amount of medical liquid accommodated before or after the above point may flow within the tube part 1240 while maintaining a set amount, and the set amount of medical liquid may be discharged to the needle assembly via the second conduit P2.

Referring to FIGS. 8, 9, 10A, 11A, and 12, the pressure-applying part 1250 according to another embodiment of the present disclosure can come into contact with the rotating part 1230, and may come into contact with and apply pressure to the tube part 1240 in response to the rotation of the rotating part 1230.

Specifically, the pressure-applying part 1250 may be connected to the rotating part 1230, specifically the connection part 1235, and the rotating plate 1233 may receive a driving force from the driving part 1210 and rotate in one direction.

As the connection part 1235, which is interlocked with the rotation of the rotating plate 1233, rotates, the pressure-applying part 1250 may come into contact with the tube part 1240 while moving in a preset direction on the connection part 1235, specifically in such a direction that the pressure-applying part 1250 is away from the center of rotation of the connection part 1235, and may compress and apply pressure to the tube part 1240.

Referring to FIGS. 8, 9, 10A, 11A, and 12, the pressure-applying part 1250 according to another embodiment of the present disclosure may include a pressure-applying body 1251 and a pushing part 1258.

The pressure-applying body 1251 is movable in a preset direction on the rotating part 1230, specifically the connection part 1235, and may come into contact with and apply pressure to the tube part 1240, may compress the tube part 1240 at the opposite side of the tube part 1240, which is supported by being brought into contact with the inner surface of the unit housing part 1231.

Referring to FIGS. 9 and 11A, the pressure-applying body 1251 according to another embodiment of the present disclosure may include a moving part 1252, the pressure-applying member 1256, and a pin part 1257.

The moving part 1252 can come into contact with the pushing part 1258 and move on the connection part 1235. The moving part 1252 is movable in such a direction that the moving part 1252 is spaced apart from the connection part 1235 by the elastic restoring force of the pushing part 1258, is spaced apart from the connection part 1235, can come into contact with the tube part 1240, and may compress and apply pressure to the tube part 1240.

Referring to FIGS. 8, 9, 10A, 11A, and 12, the pressure-applying member 1256 may be rotatably disposed on the moving part 1252, and the pin part 1257 extending along a longitudinal central axis may connect the moving part 1252 to the pressure-applying member 1256.

Accordingly, the pressure-applying member 1256 can rotate in the clockwise or counterclockwise direction with the pin part 1257 as the center axis of rotation.

Referring to FIG. 8, the pressure-applying member 1256 according to another embodiment of the present disclosure may have an outer peripheral surface formed in a curved shape, specifically, in a cylindrical shape, and may be rotatably connected to the moving part 1252.

Referring to FIG. 8, the pressure-applying member 1256 according to another embodiment of the present disclosure is formed as a single member and compresses and applies pressure to the tube part 1240 at a single point, but is not limited thereto, and various modifications are possible, such as a configuration in which a plurality of pressure-applying members 1256 are provided, and compress and apply pressure to the tube part 1240 at a plurality of points.

Referring to FIGS. 7, 8, 9, 10A, and 11A, a protrusion 1253 may be formed to protrude from the moving part 1252 according to another embodiment of the present disclosure by a certain thickness in a longitudinal direction (vertical direction based on FIG. 8), which is formed parallel to a center axis of rotation of the connection part 1235.

Specifically, referring to FIGS. 7 and 11A, the protrusion 1253 formed to protrude toward the cover part 1270 from one surface of the moving part 1252, which faces the cover part 1270, is movable on an inner side of the guide part 1271 of the cover part 1270.

Referring to FIGS. 10A to 10C, since the protrusion 1253 formed to protrude from the moving part 1252 according to another embodiment of the present disclosure moves on the guide part 1271 formed on the cover part 1270, the rotating part 1230, upon receiving a driving force from the driving part 1210, may rotate by a certain angle, and then, the pressure-applying member 1256 may move from the center of rotation of the rotating part 1230 toward the tube part 1240 in a preset region.

Referring to FIG. 11B, the pressure-applying member 1256, having moved toward the tube part 1240, may remain in a state of compression on the tube part 1240, maintained by the elastic restoring force of the pushing part 1258.

Referring to FIGS. 10A to 10C, the guide part 1271 formed in the cover part 1270 may be formed to have different distances from the center of the cover part 1270 in a preset section along the circumference of the cover part 1270 with respect to the center.

Referring to FIGS. 10A to 10C, in the guide part 1271 formed in the cover part 1270, a first section S1, a second section S2, and the first section S1 are sequentially formed along the circumference of the cover part 1270 based on the center of the cover part 1270.

Referring to FIG. 10A, the second section S2 provided in the guide part 1271 may be formed such that a distance from the center of the cover part 1270 to an inner circumferential surface of the guide part 1271 is relatively greater than that of the first section S1.

Referring to FIG. 10A, the protrusion 1253 formed to protrude toward the cover part 1270 from one surface of the moving part 1252 facing the cover part 1270 may be located in the first section S1 of the guide part 1271 formed on the cover part 1270.

Referring to FIG. 10B, as the rotating part 1230 receives a driving force from the driving part 1210 and rotates in a preset direction (clockwise direction based on FIG. 10B), the pressure-applying body 1251 connected to the connection part 1235, specifically the moving part 1252, may be rotated together, and the protrusions 1253 formed to protrude from the moving part 1252 may enter the second section S2 on the guide part 1271, which has a relatively greater distance from the center than the first section S1.

Since the second section S2 provided in the guide part 1271 is formed such that a distance from the center of the cover part 1270 to the inner circumferential surface of the guide part 1271 is relatively greater than that of the first section S1, the protrusions 1253 may move in the second section S2 of the guide part 1271 in such a direction that the protrusions 1253 is spaced apart from the connection part 1235, i.e., toward the tube part 1240.

Referring to FIG. 10C, the moving part 1252 can move toward the tube part 1240 on the connection part 1235, and the pressure-applying member 1256 provided on the moving part 1252 can come into contact with the tube part 1240 and compress and apply pressure to the tube part 1240.

Referring to FIGS. 9, 11B, and 13, the pushing part 1258 according to another embodiment of the present disclosure is disposed between the moving part 1252 and the connection part 1235, and may exhibit an elastic restoring force directed toward the tube part 1240.

Referring to FIG. 10C, the protrusion 1253 may move from the center toward the tube part 1240 after entering the second section S2 of the guide part 1271 due to the elastic restoring force of the pushing part 1258.

The moving part 1252, on which the protrusion 1253 is formed to protrude, may move in such a direction that the moving part 1252 is spaced apart from the connection part 1235 and come into contact with the tube part 1240, and may compress and apply pressure to the tube part 1240 to cause the internal cross-sectional area of the tube part 1240 to be zero.

Referring to FIG. 9, a plurality of pushing parts 1258 may be provided, and arranged side by side to be spaced apart from each other.

Referring to FIGS. 9, 12, and 13, stopper parts 1254 may be formed to protrude from the pressure-applying body 1251 according to another embodiment of the present disclosure, specifically a side surface of the moving part 1252 so as to be able to come into contact with the locking part 1260, which will be described later.

Referring to FIGS. 9, 12, and 13, the stopper parts 1254 may be formed to protrude outward from both sides of the moving part 1252 with respect to a central axis in the moving direction. As a result, a lateral region of the moving part 1252, in which the stopper part 1254 is formed to protrude, may be formed to be relatively wider than a region in which the stopper part 1254 is not formed.

Referring to FIGS. 12 and 13, an inclined part 1255 is formed at an end portion of the stopper part 1254 facing the connection part 1235 such that a width is relatively reduced based on the central axis of the moving part 1252 in the moving direction.

Due to the formation of the inclined part 1255, a bent part 1261 formed at one end portion of the locking part 1260, which will be described later, can ride along the inclined part 1255 and rest over the end portion of the stopper part 1254, and the locking part 1260 can block the moving part 1252 from moving backward toward the connection part 1235.

According to another embodiment of the present disclosure, a plurality of stopper parts 1254 are provided, and may be formed to protrude from both sides of the moving part 1252 with respect to the central axis in the moving direction. The inclined part 1255 may be formed at one end portion of each of the plurality of stopper parts 1254 facing the connection part 1235.

Referring to FIGS. 9, 12, and 13, the locking part 1260 according to another embodiment of the present disclosure is movable in a preset direction, and may come into contact with and support one surface of the pressure-applying part 1250 while the pressure-applying part 1250 compresses and applies pressure to the tube part 1240, and may fix the position of the pressure-applying part 1250.

Referring to FIGS. 9, 12, and 13, the locking part 1260 may be formed as a single member that is symmetrical on both sides with respect to a central portion thereof. The locking part 1260 may be formed of an elastically deformable material.

Referring to FIGS. 9, 12, and 13, the locking part 1260 may be formed with one side open, and the one side end portion may exhibit an elastic restoring force toward the center of the locking part 1260.

Specifically, end portions of the locking part 1260, which face each other on one side where they are open, may exhibit an elastic restoring force directed toward each other.

Referring to FIGS. 12 and 13, the locking part 1260 according to another embodiment of the present disclosure may be disposed between the pressure-applying part 1250 and the rotating part 1230.

Referring to FIGS. 9, 12, and 13, the bent part 1261, which is bent at least once, may be formed at one end portion of the locking part 1260 according to another embodiment of the present disclosure facing the pressure-applying part 1250.

The bent part 1261 formed in the locking part 1260 may be in surface contact with the pressure-applying part 1250 and may support the pressure-applying part 1250, and specifically, referring to FIG. 13, the bent part 1261 formed in the moving part 1252 may rest over the stopper part 1254 and may block the moving part 1252 from moving toward the connection part 1235.

By preventing the moving part 1252 from moving toward the connection part 1235, the pressure-applying part 1250 may continue to compress and apply pressure to the tube part 1240, thereby maintaining a state in which the internal cross-sectional area of the tube part 1240 remains zero.

Referring to FIGS. 12 and 13, both end portions of the locking part 1260, which are located on one side where they open, can come into contact with an outer peripheral surface of the stopper part 1254, which is formed to protrude from the moving part 1252, while being spaced apart from each other.

Referring to FIGS. 10C, 11B, and 13, as the moving part 1252 moves in such a direction the moving part 1252 is spaced apart from the connection part 1235, each of the facing end portions of the locking part 1260 rides along the inclined part 1255, which is formed to be inclined on one end portion of the stopper part 1254, and comes to rest on the one end portion of the stopper part 1254.

In addition, the facing end portions of the locking part 1260 exhibit an elastic restoring force in a direction that approaches each other, and thus are gathered in a direction in which a distance therebetween relatively decreases, and the bent part 1261, which is bent at least once, may be formed at the end portion of the locking part 1260 and may be in surface contact with the end portion of the stopper part 1254.

In addition, since the locking part 1260 comes into contact with and supports the end portion of the stopper part 1254, the moving part 1252 may be blocked from moving backward toward the connection part 1235 by the elastic restoring force of the tube part 1240, and the state, in which the pressure-applying part 1250 compresses and applies pressure to the tube part 1240 and the internal cross-sectional area of the tube part 1240 remains zero, may be maintained.

Referring to FIGS. 9, 12, and 13, the pushing part 1258 may be disposed between the locking part 1260 and the moving part 1252, and thus, the locking part 1260 can be fixed in position while in close contact with the rotating part 1230, specifically the connection part 1235, and the moving part 1252 can be pushed toward the tube part 1240 due to the elastic restoring force of the pushing part 1258.

The medical liquid injection device according to another embodiment of the present disclosure, except for the configuration of the medical liquid discharge assembly 1200, has the same configuration, operating principle, and effects of the housing 5, the attachment part 6, the body part 10, the reservoir 20, the needle assembly 30, the battery 40, and the control module as those of the medical liquid injection device 1000 according to an embodiment of the present disclosure, and thus detailed descriptions of these components are omitted to the extent of overlapping.

In the medical liquid discharge assembly according to embodiments of the present disclosure, the position of the pressure-applying part can be fixed by the locking part coming into contact with and supporting one surface of the pressure-applying part in a state in which the priming operation is completed and the pressure-applying part applies pressure to the tube part.

In addition, by fixing the position of the pressure-applying part, the internal cross-sectional area of the tube part can remain zero, and changes in the degree of compression due to the elastic restoring force of the tube part can be prevented.

Hereinafter, a medical liquid discharge assembly and a medical liquid injection device including the same according to another embodiments of the present disclosure will be described.

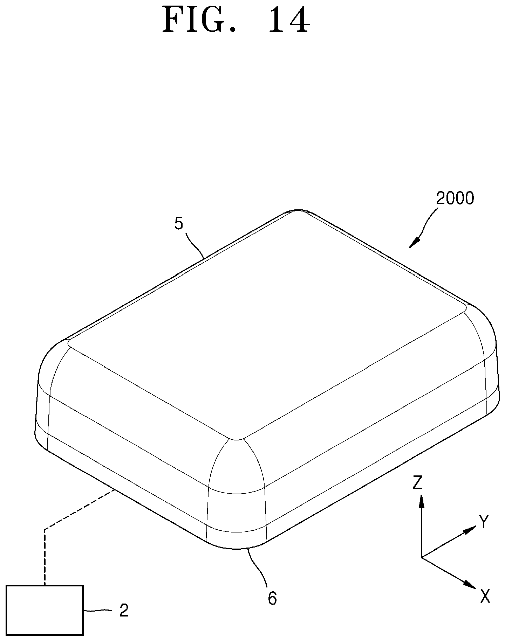

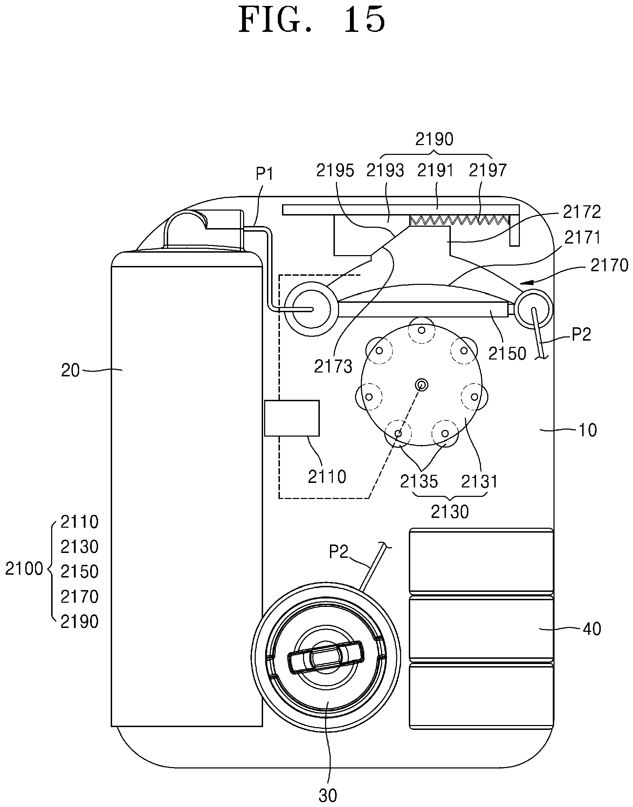

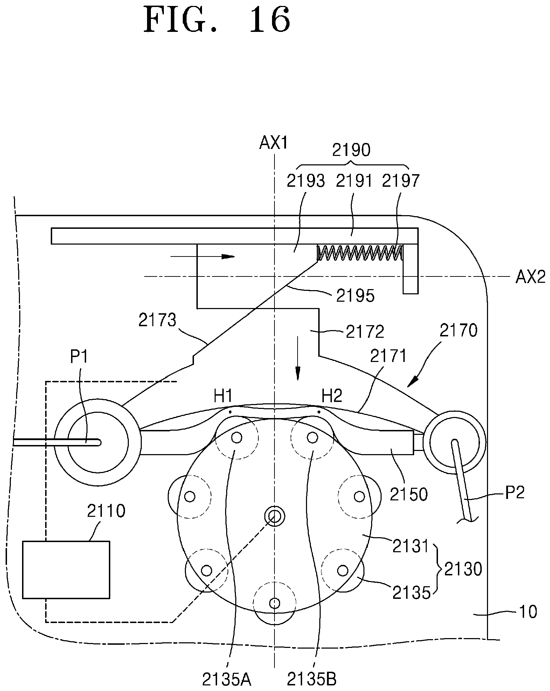

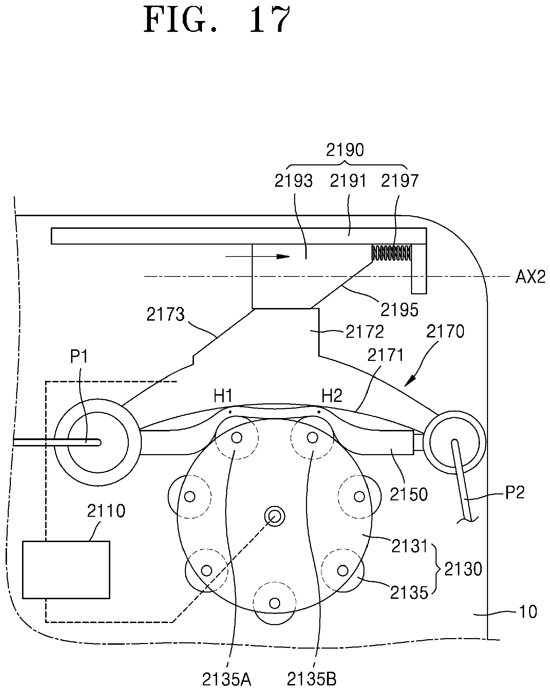

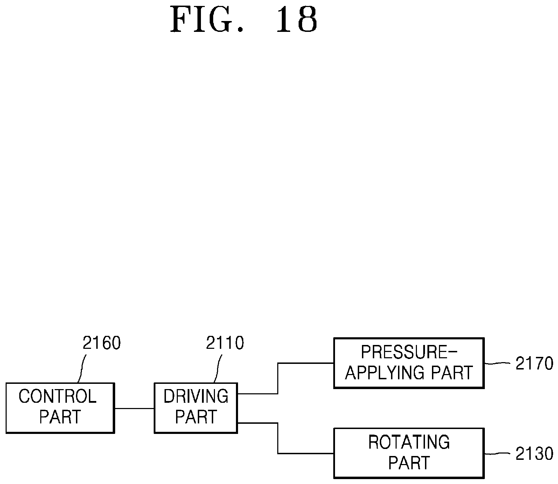

FIG. 14 is a perspective view illustrating a medical liquid injection device according to an embodiment of the present disclosure. FIG. 15 is a plan view illustrating an interior of the medical liquid injection device according to an embodiment of the present disclosure. FIGS. 16 and 17 are enlarged views partially illustrating a locking part, a rotating part, and a pressure-applying part according to an embodiment of the present disclosure. FIG. 18 is a block diagram illustrating a control part according to an embodiment of the present disclosure.

Referring to FIG. 14, a medical liquid injection device 2000 according to an embodiment of the present disclosure is attached to a medical liquid injection subject, and may inject a preset amount of medical liquid stored therein to a user.

In an optional embodiment, the medical liquid injection device 2000 may be mounted on the body of the medical liquid injection subject (hereinafter referred to as the "user").

In an optional embodiment, the medical liquid injection device 2000 may be mounted on an animal to inject a medical liquid.

The medical liquid injection device 2000 according to an embodiment of the present disclosure may be used for various purposes depending on the type of medical liquid to be injected. For example, the medical liquid may include an insulin-based medical liquid for a diabetic patient, and may include a medical liquid for other pancreas, a medical liquid for heart, and other various types of medical liquids.

Referring to FIG. 14, the medical liquid injection device 2000 may be connected to a remote device 2 that is wired or wirelessly connected.

A user may manipulate the remote device 2 to use the medical liquid injection device 2000 and monitor the state of use of the medical liquid injection device 2000. For example, the amount of medical liquid injected from the medical liquid injection device 2000, the number of injections of the medical liquid, the amount of medical liquid stored in a reservoir 20, user's bio information, and the like may be monitored, and based on this, the user may drive the medical liquid injection device 2000.

The remote device 2 according to an embodiment of the present disclosure can remotely transmit and receive signals to and from a control part 2160 to be described later, and the control part 2160 may receive signals from the external remote device 2 and transmit electrical signals to a driving part 2110 to be described later.

The driving part 2110, upon receiving the electrical signals from the control part 2160, may transmit a driving force to a rotating part 2130 and a pressure-applying part 2170, which will be described later, to rotationally drive the rotating part 2130, and move the pressure-applying part 2170 in the direction of a preset first axis AX1.

In the present specification, the term "remote device 2" refers to a communication terminal that may use an application in a wired or wireless communication environment. Here, the remote device 2 may be a user's portable terminal.

In more detail, the remote device 2 may include a computer (e.g., desktop, laptop, tablet, or the like), a media computing platform (e.g., cable, satellite set-top box, digital video recorder), a handheld computing device (e.g., personal digital assistant (PDA), e-mail client, or the like), a mobile phone in any form, a form of a wearable device that can be attached or mounted on the user's body, or any form of another kind of computing or communication platform, but the present disclosure is not limited thereto.

The medical liquid injection device 2000 and the remote device 2 may communicate through a communication network. In this case, the communication network refers to a communication network providing an access path so that the remote device 2 may transmit and receive data after accessing a service server (not shown in the drawings). The communication network may include, for example, a wired network such as LANs, WANs, MANs, and ISDNs, and a wireless network such as wireless LANs, a CDMA network, a Bluetooth network, and a satellite communication network, but the scope of the present disclosure is not limited thereto.

Referring to FIG. 14, the remote device 2 according to an embodiment of the present disclosure is illustrated as a single device, but is not limited thereto, and may include a plurality of devices capable of communicating with the medical liquid injection device 2000.

Referring to FIG. 14, the remote device 2 is illustrated as a single device, but the present disclosure is not necessarily limited thereto, and may include a plurality of devices capable of communicating with the medical liquid injection device 2000.

Referring to FIG. 14, the medical liquid injection device 2000 according to an embodiment of the present disclosure includes a housing 5 that forms an exterior and has a hollow interior, and an attachment part 6 that is disposed on one side (a lower side based on FIG. 14) of the housing 5 and is attached to the skin of a user.

A plurality of components may be disposed in an interior space of the housing 5 according to an embodiment of the present disclosure. Referring to FIGS. 14 and 15, the medical liquid injection device 2000 according to an embodiment of the present disclosure may include the housing 5, the attachment part 6, a body part 10, a medical liquid discharge assembly 2100, and a reservoir 20, a needle assembly 30, and a battery 40.

The body part 10 is disposed inside the housing 5, and the reservoir 20, the needle assembly 30, the battery 40, and the medical liquid discharge assembly 2100 may be disposed on the body part 10.

The body part 10 may have conducting wires installed thereon to facilitate electrical connections to the driving part 2110 to be described later and the control part 2160, for example, the body part 10 may be formed as a printed circuit board (PCB).

The reservoir 20 stores a medical liquid to be injected, and may be connected to the medical liquid discharge assembly 2100. Specifically, the reservoir 20 may be connected to the medical liquid discharge assembly 2100 through a first conduit P1.

Referring to FIG. 15, the reservoir 20 according to an embodiment of the present disclosure is disposed on the body part 10 disposed inside the housing 5, but is not limited thereto, and various modifications are possible, such as a configuration in which the reservoir 20 is installed on the outside of the housing 5, within the technical idea of supplying a medical liquid to the medical liquid discharge assembly 2100.

The needle assembly 30 is disposed inside the housing 5, and may be disposed on one side of the body part 10. The needle assembly 30 is inserted into the skin of the user, so that the discharged medical liquid may be injected into the user.

The medical liquid discharge assembly 2100 may be installed between the needle assembly 30 and the reservoir 20 according to an embodiment of the present disclosure. Specifically, the needle assembly 30 may be connected to the reservoir 20 through a tube part 2150 and a second conduit P2, which are provided in the medical liquid discharge assembly 2100.

A medical liquid may be distributed in a preset amount or at a preset frequency from the medical liquid discharge assembly 2100 according to an embodiment of the present disclosure and discharged to the needle assembly 30 via the second conduit P2.

Referring to FIGS. 15 to 18, the medical liquid discharge assembly 2100 according to an embodiment of the present disclosure is disposed inside the housing 5 and disposed on the body part 10, and may include the driving part 2110, the rotating part 2130, the tube part 2150, the control part 2160, the pressure-applying part 2170, and a locking part 2190.

Referring to FIG. 15, the battery 40 according to an embodiment of the present disclosure may supply electricity to the medical liquid injection device 2000 to activate each component. While a single battery 40 is illustrated in the drawings, the battery 40 is not limited thereto, and may be set in various ways depending on the capacity, scope of use, duration of use, or the like of the medical liquid injection device 2000.

The battery 40 according to an embodiment of the present disclosure may be disposed adjacent to the driving part 2110 and may supply electricity to the driving part 2110. In addition, the battery 40 may be connected to the control part 2160, and based on the electrical signals measured by a sensor unit, data about the number of rotations or rotational speed of the driving part 2110, the amount of medical liquid stored in the reservoir 20, the amount of medical liquid injected into the user, and the like may be measured.

Referring to FIGS. 15 and 18, the driving part 2110 according to an embodiment of the present disclosure is movable in a preset direction, and may generate a driving force and transmit the driving force to the rotating part 2130 and the pressure-applying part 2170.

The driving part 2110 according to an embodiment of the present disclosure may be electrically connected to the control part 2160, which will be described later, and can perform rotational and linear motions upon receiving electrical signals from the control part 2160.

In an optional embodiment, the driving part 2110 may be driven by direct manipulation by the user, and the user may externally apply a force to the driving part 2110 to perform a rotational motion and a linear motion.

The driving part 2110 according to an embodiment of the present disclosure may be disposed in the interior space of the housing 5 and may be disposed on the body part 10. The driving part 2110 may transmit a driving force to the medical liquid discharge assembly 2100.

In an optional embodiment, the driving part 2110 and the medical liquid discharge assembly 2100 may each be disposed as a separate component, may be configured as components that constitute, together with the driving part 2110, the medical liquid discharge assembly 2100, according to an embodiment of the present disclosure.

In an optional embodiment, the driving part 2110 may include a drive shaft (not shown in the drawings) and a drive piece (not shown in the drawings), and the drive shaft may repeatedly move forward and backward while performing a linear reciprocating motion in a preset direction and may transmit a driving force to the drive piece connected to the drive shaft to cause the drive piece to perform a linear reciprocating motion.

The driving part 2110 according to an embodiment of the present disclosure may be any type of device that uses electricity to generate both suction and discharge forces for the medical liquid. For example, all types of pumps such as a mechanical displacement type micropump and an electromagnetic motion type micropump may be used.

The mechanical displacement type micropump is a pump that uses solid or fluid motion such as a gear or diaphragm to generate a pressure difference to induce fluid flow, and includes a diaphragm displacement pump, a fluid displacement pump, a rotary pump, and the like. The electromagnetic motion micropump is a pump that directly uses electrical or magnetic energy for fluid movement, and may include an electro-hydrodynamic pump (EHD), an electro-osmotic pump, a magneto-hydrodynamic pump, an electro-wetting pump, and the like.

Referring to FIGS. 15 and 18, the driving part 2110 according to an embodiment of the present disclosure may transmit a driving force to the rotating part 2130 and rotate the rotating part 2130 in a preset direction.

The rotating part 2130, specifically a rotating body 2131, which receives the driving force from the driving part 2110, may rotate in a preset direction, either in a clockwise or counterclockwise direction, around the center.

The driving part 2110 according to an embodiment of the present disclosure may be directly or indirectly connected to the pressure-applying part 2170, which will be described later, and may transmit a driving force to the pressure-applying part 2170 to move the pressure-applying part 2170 in the direction of the preset first axis AX1 (in the downward direction from the upper side based on FIG. 16).

However, the present disclosure is not limited thereto, and various modifications are possible, such as a configuration in which the pressure-applying part 2170 may be directly or indirectly connected to the rotating part 2130, and the driving part 2110 transmits a driving force to the rotating part 2130 to cause the rotating part 2130 to rotate in one direction, thereby moving the pressure-applying part 2170 in the direction of the first axis AX1 (in the downward direction from the upper side based on FIG. 16).

Referring to FIG. 18, the driving part 2110 according to an embodiment of the present disclosure may be electrically connected to the control part 2160, which will be described later, and may be driven upon receiving an electrical signal from the control part 2160.

Referring to FIGS. 15 to 17, the rotating part 2130 according to an embodiment of the present disclosure can come into contact with the driving part 2110 and can rotate in one direction in response to the movement of the driving part 2110.

Referring to FIGS. 15 to 17, the rotating part 2130 according to an embodiment of the present disclosure may include the rotating body 2131 and a contact part 2135.

The rotating body 2131 according to an embodiment of the present disclosure can rotate in one direction around a preset center, which may be fixed in position to the body part 10. The rotating body 2131 may receive a driving force from the driving part 2110 and rotate in one direction.

Referring to FIGS. 15 to 18, the rotating body 2131 according to an embodiment of the present disclosure may receive a driving force from the driving part 2110, which is driven upon receiving an electrical signal from the control part 2160, and rotate in one direction.

Referring to FIGS. 15 to 17, the rotating body 2131 according to an embodiment of the present disclosure may be disposed to face the tube part 2150.

Referring to FIGS. 15 to 17, the contact part 2135 according to an embodiment of the present disclosure is connected to the rotating body 2131, and may be disposed to be rotatable on the rotating body 2131. A plurality of contact parts 2135 may be provided. The plurality of contact parts 2135 may be arranged equidistantly around the center of the rotating body 2131.

Referring to FIGS. 15 to 17, according to an embodiment of the present disclosure, seven contact parts 2135 may be provided and arranged equidistantly around the center of the rotating body 2131, but the present disclosure is not limited thereto, and various modifications are possible within the technical idea in which at least two contact parts 2135 are in contact with the tube part 2150, and the tube part 2150 is compressed by the pressure-applying part 2170, which will be described later, and the contact parts 2135, and has a zero cross-sectional area.

Referring to FIGS. 15 to 17, the contact part 2135 according to an embodiment of the present disclosure may have an outer peripheral surface formed as a curved surface. Specifically, the contact part 2135 is formed in a cylindrical shape, and may be rotatably disposed on the rotating body 2131.

Referring to FIG. 17, according to an embodiment of the present disclosure, a pair of contact parts 2135A and 2135B are each in contact with the tube part 2150 at a pair of contact points H1 and H2, and at the contact points H1 and H2, the tube part 2150 is compressed by the pressure-applying part 2170 and the contact part 2135 to have a zero cross-sectional area, this allows a set amount of medical liquid to be set based on the internal volume of the tube part 2150 between the pair of contact points H1 and H2.

As the rotating part 2130 rotates upon receiving a driving force from the driving part 2110, a set amount of medical liquid flows within the tube part 2150 via the second conduit P2, and may be discharged into the needle assembly 30.

In an optional embodiment, the amount of medical liquid accommodated between the pair of contact points may be set differently by varying the number of contact parts 2135. For example, when the number of contact parts 2135 is increased, the amount of the distributed medical liquid may be set to be relatively small, and the medical liquid finally discharged may be injected in a set amount.

Referring to FIGS. 15 to 17, the contact part 2135 according to an embodiment of the present disclosure may include a pin, and the pin may be connected to the rotating body 2131. Accordingly, the contact part 2135 may be rotated in the clockwise or counterclockwise direction on the rotating body 2131 with the pin as the center axis of rotation.

By rotatably connecting the contact part 2135 according to an embodiment of the present disclosure to the rotating body 2131, the contact part 2135 can be rotated together with the rotating body 2131 when the rotating body 2131 rotates upon receiving a driving force from the driving part 2110 while the tube part 2150 is compressed, which ensures the stable flow of the medical liquid accommodated inside the tube part 2150, specifically, between the pair of contact points H1 and H2.

Referring to FIGS. 15 to 17, the tube part 2150 according to an embodiment of the present disclosure may be formed of a flexible material and may be disposed between the rotating part 2130 and the pressure-applying part 2170 to be described later.

The tube part 2150 according to an embodiment of the present disclosure may be formed with a hollow interior, through which the medical liquid may flow, and may be disposed adjacent to the rotating part 2130.

Referring to FIGS. 15 to 17, the tube part 2150 according to an embodiment of the present disclosure can come into contact with the pressure-applying part 2170 and the rotating part 2130 from both sides, and the tube part 2150 may be compressed by the pressure-applying part 2170 and the rotating part 2130.

Referring to FIGS. 15 to 17, both end portions of the tube part 2150 according to an embodiment of the present disclosure may be fixed in position to the pressure-applying part 2170. One end portion of the tube part 2150 may be connected to the first conduit P1, and may receive a medical liquid from the reservoir 20 via the first conduit P1.

Another end portion of the tube part 2150, which is opposite to one end portion connected to the first conduit P1, may be connected to the second conduit P2. The tube part 2150 may supply a medical liquid to the needle assembly 30 via the second conduit P2, and the medical liquid may be discharged to the user through the needle assembly 30.

As the pressure-applying part 2170 moves toward the rotating part 2130 with both end portions of the tube part 2150 fixed in position to the pressure-applying part 2170, the tube part 2150 may move, in conjunction with the pressure-applying part 2170, toward the rotating part 2130 (in the downward direction from the upper side based on FIG. 16).

Referring to FIGS. 16 and 17 as the pressure-applying part 2170 moves toward the rotating part 2130, the tube part 2150 connected to the pressure-applying part 2170 approaches the rotating part 2130, specifically the contact part 2135, and as the pressure-applying part 2170 moves further while the tube part 2150 is in contact with the contact part 2135, the tube part 2150 may be compressed between the pressure-applying part 2170 and the rotating part 2130.

Referring to FIG. 17, the tube part 2150 may be compressed at a plurality of points. Specifically, the tube part 2150 may be in contact with and compressed at the pair of contact points H1 and H2 by the pair of contacts parts 2135A and 2135B, and may have a zero internal cross-sectional area at the pair of contact points H1 and H2.

Since the internal cross-sectional area of the tube part 2150 becomes zero at the pair of contact points H1 and H2, a preset amount of medical liquid may be accommodated in the interior space of the tube part 2150 between the pair of contact points H1 and H2, and as the rotating part 2130 rotates upon receiving a driving force from the driving part 2110, the medical liquid may flow within the tube part 2150 as the plurality of contact parts 2135 push the tube part 2150.

As the rotating part 2130 rotates, the plurality of contact parts 2135, which are rotatably connected to the rotating body 2131, may cause the medical liquid accommodated inside the tube part 2150 to flow into the needle assembly 30 by a preset amount via the second conduit P2.

Referring to FIGS. 15 to 17, since the tube part 2150 according to an embodiment of the present disclosure includes a flexible material and can be deformed in shape, a curved section with a preset radius of curvature may be formed in the tube part 2150 as the pressure-applying part 2170 moves in a direction toward the rotating part 2130 and is in contact with the tube part 2150 and the rotating part 2130, specifically, the contact part 2135.

Referring to FIG. 17, a curved surface part 2171 may be formed on one surface (a lower surface based on FIG. 17) of the pressure-applying part 2170, which faces the tube part 2150, to correspond to a curved section formed when the tube part 2150 is in contact with and compressed by the contact part 2135.

In addition, since the tube part 2150 includes a flexible material, the tube part 2150 can be in contact with, compressed by, and deformed in shape by the rotating part 2130, specifically the contact part 2135 and the pressure-applying part 2170, and the internal cross-sectional area of the tube part 2150 becomes zero at the pair of contact points H1 and H2.

In addition, since the internal cross-sectional area of the tube part 2150 becomes zero at the pair of contact points H1 and H2, a set amount of medical liquid accommodated between the pair of contact points H1 and H2 may flow within the tube part 2150 while maintaining a set amount, and the set amount of medical liquid may be discharged to the needle assembly 30 via the second conduit P2.

Referring to FIG. 18, the control part 2160 according to an embodiment of the present disclosure may control overall operations of the medical liquid injection device 2000. The control part 2160 is electrically in contact with and connected to internal devices such as the driving part 2110, the battery 40, and a plurality of sensor units, and may control operations thereof.

Based on data measured by an encoder unit (not shown in the drawings), the control part 2160 according to an embodiment of the present disclosure may calculate a rotation angle and a rotation speed of the driving part 2110 and the rotating part 2130 that rotates upon receiving a driving force from the driving part 2110, and calculate a movement distance of the plunger, which is provided in the reservoir 20 and configured to discharge the medical liquid, and the amount of medical liquid discharged.

The control part 2160 according to an embodiment of the present disclosure can transmit and receive signals to and from the remote device 2, and the control part 2160 may receive signals from the external remote device 2 and transmit electrical signals to the driving part 2110.

The control part 2160 according to an embodiment of the present disclosure may transmit an electrical signal to the driving part 2110 to drive the driving part 2110, and the rotating part 2130 may be rotated in one direction in response to the movement of the driving part 2110.

The control part 2160 according to an embodiment of the present disclosure may transmit an electrical signal to the driving part 2110 to drive the driving part 2110, and the driving part 2110 may transmit a driving force to the pressure-applying part 2170, which will be described later, to move the pressure-applying part 2170 in the direction of the first axis AX1.

In an optional embodiment, various modifications are possible, such as a configuration in which the pressure-applying part 2170, which will be described later, may be connected to the rotating part 2130, and as the rotating part 2130 is rotated in one direction in response to the movement of the driving part 2110, the pressure-applying part 2170 may be moved in the direction of the first axis AX1 in conjunction with the rotation of the rotating part 2130.

Referring to FIGS. 15 to 18, the pressure-applying part 2170 according to an embodiment of the present disclosure is disposed to face the rotating part 2130 with the tube part 2150 interposed therebetween and movable in the direction of the first axis AX1, and the pressure-applying part 2170 may, in conjunction with the rotating part 2130, come into contact with and apply pressure to the tube part 2150 from both sides.

Referring to FIGS. 15 to 17, the tube part 2150 may be connected to the pressure-applying part 2170 according to an embodiment of the present disclosure, and specifically, both end portions of the tube part 2150 may be connected to a plurality of points of the pressure-applying part 2170.

Since both end portions of the tube part 2150 are connected to the plurality of points of the pressure-applying part 2170, the tube part 2150 is disposed in a straight shape when not in contact with the contact part 2135, but may form a curved section when in contact with the contact part 2135 and in contact with and compressed by the pressure-applying part 2170 and the rotating part 2130.

Referring to FIGS. 15 to 17, the curved surface part 2171 may be formed on one surface, specifically the lower surface (based on FIG. 15) of the pressure-applying part 2170 according to an embodiment of the present disclosure, which faces the tube part 2150, to have a certain curvature to correspond to the curved section.

As a result, it is possible to correspond to the curved section formed in the tube part 2150 that is in contact with and compressed by the pressure-applying part 2170 and the rotating part 2130, and the tube part 2150 may stably remain in the compressed state.

Referring to FIG. 15, a protrusion 2172 may be formed to protrude outward from the pressure-applying part 2170 according to an embodiment of the present disclosure. Specifically, the protrusion 2172 may be formed to protrude in a direction toward the locking part 2190, which will be described later, and may be disposed to be in contact with a locking body 2193 provided in the locking part 2190.

Referring to FIGS. 15 to 17, the protrusion 2172 according to an embodiment of the present disclosure may have a second inclined surface 2173 formed on one side that comes into contact with the locking part 2190, specifically the locking body 2193. The second inclined surface 2173 formed on the protrusion 2172 is in contact with a first inclined surface 2195 formed on the locking body 2193, and may be formed in a shape corresponding to the shape of the first inclined surface 2195.

Since the second inclined surface 2173 formed on the protrusion 2172 and the first inclined surface 2195 formed on the locking body 2193 are in contact with each other, the locking body 2193 may move along the second inclined surface 2173 formed on the protrusion 2172, and as the pressure-applying part 2170 moves in the direction of the first axis AX1, the locking body 2193 may stably move in the direction of a second axis AX2 that forms a predetermined angle with the first axis AX1.

Referring to FIGS. 15 to 18, the pressure-applying part 2170 according to an embodiment of the present disclosure is movable in a preset direction, specifically, in the direction of the first axis AX1 upon receiving a driving force from the driving part 2110.

However, the present disclosure is not limited thereto, and the pressure-applying part 2170 is directly or indirectly connected to the rotating part 2130, and when the rotating part 2130 rotates in one direction upon receiving a driving force from the driving part 2110, in conjunction with the rotation of the rotating part 2130, the pressure-applying part 2170 may move in the direction of the first axis AX1 by a preset movement distance.

In the present disclosure, the term "movement distance" refers to a vertical distance d formed along the first axis AX1 with the second inclined surface 2173 as the hypotenuse, and as the pressure-applying part 2170 is moved closer to the rotating part 2130 by the movement distance, a curved section may be formed in the tube part 2150 disposed between the pressure-applying part 2170 and the rotating part 2130, and the tube part 2150 can come into contact with the rotating part 2130, specifically the contact part 2135 and may be compressed by the contact part 2135 and the curved surface part 2171 formed on the pressure-applying part 2170.

Referring to FIGS. 16 and 17, the tube part 2150 is in contact with and compressed at the pair of contact points H1 and H2 by the pressure-applying part 2170 and the rotating part 2130, specifically, a plurality of contact parts 2135, and thus, may have a zero internal cross-sectional area.

As a result, a set amount of medical liquid may be accommodated inside the tube part 2150 between the plurality of points H1 and H2 at which the tube part 2150 is compressed by the pressure-applying part 2170 and the contact part 2135, and as the rotating part 2130 receives a driving force from the driving part 2110 and rotates, the set amount of medical liquid may flow within the tube part 2150 and may be discharged into the needle assembly 30 via the second conduit P2.

Referring to FIGS. 15 to 17, the locking part 2190 according to an embodiment of the present disclosure is disposed to be in contact with the pressure-applying part 2170, is movable in the direction of the second axis AX2 that forms a certain angle with the first axis AX1, and can be located on a movement path of the pressure-applying part 2170.

Referring to FIGS. 15 to 17, the locking part 2190 according to an embodiment of the present disclosure may include a locking base part 2191, the locking body 2193, and an elastic member 2197.

Referring to FIGS. 15 to 17, the locking base part 2191 according to an embodiment of the present disclosure is disposed to be spaced apart from the pressure-applying part 2170, and may be installed on the body part 10. The locking base part 2191 may be fixed in position to the body part 10.

Referring to FIGS. 15 to 17, the locking base part 2191 may provide a movement path for the locking body 2193, and an elastic member 2197 may be connected thereto.

The locking body 2193 according to an embodiment of the present disclosure is movable on the locking base part 2191, and specifically, the locking body 2193 is movable in the direction of the second axis AX2 (in a left-right direction based on FIG. 17) that forms a certain angle with the first axis AX1.

The second axis AX2, which is the axis of movement for the locking body 2193 according to an embodiment of the present disclosure, may be formed perpendicular to the first axis AX1, which is the axis of movement for the pressure-applying part 2170.

As a result, by moving the pressure-applying part 2170 in the direction of the first axis AX1, and disposing the locking body 2193 on the movement path of the pressure-applying part 2170 in the direction of the second axis AX2, which is perpendicular to the first axis AX1, the pressure-applying part 2170 may be blocked from moving back to a position prior to moving in the direction of the first axis AX1.

Referring to FIG. 17, the pressure-applying part 2170 according to an embodiment of the present disclosure moves in the direction of the first axis AX1, and, in conjunction with the rotating part 2130, comes into contact with and compresses the tube part from both sides (in the vertical direction based on FIG. 17), and at the preset contact points H1 and H2, the internal cross-sectional area of the tube part 2150 becomes zero.

At this time, the tube part 2150, which includes a flexible material, exhibits an elastic restoring force in the direction before being compressed, that is, in the direction of increasing the internal cross-sectional area, and there is a problem that the pressure-applying part 2170 may be moved to the initial position along the first axis AX1 due to the elastic restoring force.

Referring to FIG. 17, the pressure-applying part 2170 is moved in the direction of the first axis AX1 by a certain distance d, and the locking body 2193 is moved along the second axis AX2 perpendicular to the first axis AX1 while in contact with the protrusion 2172 formed on the pressure-applying part 2170, and is disposed on the movement path of the pressure-applying part 2170, specifically, on the first axis AX1, which allows the pressure-applying part 2170 to be blocked from moving to the initial position due to the elastic restoring force of the tube part 2150.

Referring to FIG. 17, as the locking body 2193 blocks the movement of the pressure-applying part 2170, the compressed state of the tube part 2150, which is achieved by the pressure-applying part 2170 and the rotating part 2130, specifically the contact part 2135, may be maintained, this ensures the internal cross-sectional area of the tube part 2150 remains zero at the contact points H1 and H2.

In addition, the internal cross-sectional area of the tube part 2150 remains zero at the pair of contact points H1 and H2, allowing a set amount of medical liquid accommodated between the pair of contact points H1 and H2 to flow within the tube part 2150 and be discharged into the needle assembly 30.

Referring to FIGS. 15 to 17, the first inclined surface 2195 may be formed on one surface of the locking body 2193 according to an embodiment of the present disclosure while forming a certain angle with the second axis AX2.

Specifically, the first inclined surface 2195 may be formed at an angle forming an acute angle with the second axis AX2, and can come into contact with the second inclined surface 2173 formed on the pressure-applying part 2170 facing the locking body 2193, specifically, one surface of the protrusion 2172 and can move while coming into contact with the second inclined surface 2173.

Since the first inclined surface 2195 is formed on the locking body 2193 and can come into contact with the second inclined surface 2173, which is formed on one surface of the protrusion 2172 formed to protrude from the pressure-applying part 2170, at the same time as the pressure-applying part 2170 moves toward the rotating part 2130 in the direction of the first axis AX1, the locking body 2193 can stably move along the second inclined surface 2173 in the direction of the second axis AX2.

Referring to FIGS. 15 to 17, the elastic member 2197 according to an embodiment of the present disclosure connects the locking base part 2191 to the locking body 2193, and may be formed of an elastically deformable material.

Referring to FIGS. 15 to 17, one end portion (a left-side end portion based on FIG. 15) of the elastic member 2197 according to an embodiment of the present disclosure may be connected to the locking body 2193, and another end portion (a right-side end portion based on FIG. 15) thereof, which is opposite to the one end portion, may be connected to the locking base part 2191.

Referring to FIGS. 15 to 17, the elastic member 2197 according to an embodiment of the present disclosure may be formed as a coil-shaped spring. The elastic member 2197 may exhibit an elastic restoring force in the direction of the second axis AX2.

Specifically, the elastic member 2197 may exhibit an elastic restoring force in a direction (from a leftward direction to a rightward direction based on the FIG. 15) in which the locking body 2193 moves in the direction of the second axis AX2 along the second inclined surface 2173, which is provided on one surface of the protrusion 2172 formed to protrude from the pressure-applying part 2170.

In other words, the elastic member 2197 connecting the locking base part 2191 to the locking body 2193 may exhibit an elastic restoring force in the direction of decreasing a length thereof.

As a result, when the pressure-applying part 2170 moves in the direction of the first axis AX1, the movement path for the locking body 2193 may be formed along the second axis AX2, which forms a certain angle with the first axis AX1, specifically perpendicular to the first axis AX1, and the locking body 2193 may be moved by the elastic restoring force of the elastic member 2197.

When the locking body 2193 moves along the second axis AX2, the locking body 2193 is located between the locking base part 2191 and the pressure-applying part 2170, specifically the protrusion 2172, and the location of the locking body 2193 corresponds to the movement path of the pressure-applying part 2170 formed along the first axis AX1.

By disposing the locking body 2193 on the movement path of the pressure-applying part 2170, the return of the pressure-applying part 2170 to the original position thereof in the direction of the first axis AX1 may be blocked.

In other words, the pressure-applying part 2170 moves in the direction of the first axis AX1 by the certain distance d, and the tube part 2150 is in contact with and compressed by the pressure-applying part 2170 and the rotating part 2130, specifically, the contact part 2135, and in this case, due to the elastic restoring force of the tube part 2150, which includes a flexible material, there is a risk that the pressure-applying part 2170 may be pushed and moved to the initial position thereof in the direction of the first axis AX1, but by disposing the locking body 2193 in the movement path of the pressure-applying part 2170, the pressure-applying part 2170 may be prevented from moving.

In addition, it is possible to maintain the state in which the pressure-applying part 2170 and the rotating part 2130 are in contact with and compress the tube part 2150.

The operating principle and effects of the above-described medical liquid injection device 2000 according to an embodiment of the present disclosure will be described.

### <Medical liquid storage operation>

Referring to FIG. 15, a user injects a medical liquid into the reservoir 20 of the medical liquid injection device 2000 by using an external medical liquid injector (not shown).

The user puts the medical liquid to be injected into the medical liquid injector (not shown), and inserts the medical liquid injector into an inlet end of the reservoir 20. At this time, air priming may be performed for air remaining inside the reservoir 20.

In detail, during the assembly process of the reservoir 20, air remains inside the reservoir 20. When the medical liquid is injected while the air remains in the reservoir 20, there is a risk of injecting the air to the user together, and thus an operation (priming operation) for removing the air is required.

When the medical liquid begins to flow into the reservoir 20 from the medical liquid injector, gases, which have entered and remained inside the reservoir 20, can be pushed out of a needle cover assembly (not shown in the drawings) and discharged.

Gases that have passed through the needle cover assembly may be discharged to the outside. As a result, the gases remaining inside the reservoir 20 can be quickly discharged to the outside, allowing the gases in the reservoir 20 to be removed.

Referring to FIG. 14, by notifying the user of the fact that the medical liquid is stored in the reservoir 20 by a preset reference amount through the external remote device 2 or the like, the user may be notified in advance to use the medical liquid injection device 2000.

### <Attachment operation>

When the medical liquid is stored in the reservoir 20, the medical liquid injection device 2000 is attached to the user. Since the gases in the reservoir 20 are removed (the priming operation is completed) through the needle cover assembly in the above-described medical liquid storage operation, the needle cover assembly is removed from the medical liquid injection device 2000.

The user attaches the medical liquid injection device 2000 thereto, rotates the needle assembly 30, and inserts a needle and a cannula into the skin. The needle is inserted into the skin together with the cannula, and may induce the cannula to be inserted into the skin.

Thereafter, the needle is withdrawn from the skin, but the state in which the needle and the cannula are connected is maintained. When the user further rotates the needle assembly 30, the needle moves upward while the cannula is inserted into the skin. At least a portion of each of the cannula and the needle is connected to each other, and a path through which the medical liquid moves is formed and maintained.

### <Medical liquid injection operation>

The driving part 2110 may be driven substantially simultaneously with the operation of inserting the cannula and the needle into the user. The medical liquid injection device 2000 may inject the medical liquid into the user according to a set period and injection amount.

The user may rotate the needle assembly 30 to drive the driving part 2110 in order to insert the needle and the cannula into the skin. As the driving part 2110 is driven, the reservoir 20, specifically the plunger (not shown in the drawings) movably disposed inside the reservoir 20, may move, and the medical liquid may be discharged into the needle.

Accordingly, the medical liquid may be injected into the user according to a set driving period and speed of the driving part 2110.

Although not shown in the drawings, as the driving part 2110 is driven, the encoder unit (not shown in the drawings) may measure the rotation angle, rotation speed, or the like of the driving part 2110.

The encoder unit may measure data related to the rotation of the driving part 2110 by measuring an electrical connection signal and/or an electrical release signal. The control part 2160 may calculate a rotation angle and a rotation speed of the driving part 2110 based on the data measured by the encoder unit, and may calculate a movement distance of the plunger provided in the reservoir 20 and a discharge amount of the medical liquid based on the rotation angle and the rotation speed.

Referring to FIGS. 15 to 17, as the driving part 2110 according to an embodiment of the present disclosure is driven, the rotating part 2130 is rotated in one direction, and specifically, the rotating body 2131 is rotated around a preset center, and the plurality of contact parts 2135 may be rotatably connected to the rotating body 2131.

The plurality of contact parts 2135 may be equidistantly arranged around the circumference of the rotating body 2131, centered on the center of rotation.

Referring to FIG. 16, the pressure-applying part 2170 according to an embodiment of the present disclosure moves along the first axis AX1 in the direction toward the rotating part 2130 whose center of rotation is fixed in position.

In the present disclosure, the pressure-applying part 2170 receives a driving force from the driving part 2110 and moves along the first axis AX1 in a direction close to the rotating part 2130, but the present disclosure is not limited thereto, and various modifications are possible, such as a configuration in which the pressure-applying part 2170 is directly or indirectly connected to the rotating part 2130, which rotates in one direction in response to the movement of the driving part 2110, and moves in the direction of the first axis AX1 in conjunction with the rotation of the rotating part 2130. rotating part 2130.

Referring to FIG. 16, as the pressure-applying part 2170 moves along the first axis AX1 in a direction approaching the rotating part 2130, the tube part 2150 disposed between the pressure-applying part 2170 and the rotating part 2130 may be in contact with the rotating part 2130, specifically the contact part 2135.

Referring to FIGS. 16 and 17, the tube part 2150 may be in contact with and compressed from both sides by the contact part 2135 and the pressure-applying part 2170, and compressed at the pair of contact points H1 and H2 to have a zero internal cross-sectional area.

As a result, a set amount of medical liquid, which is preset equal to the internal volume of the tube part 2150, may be accommodated between the pair of contact points H1 and H2, and may be transferred and discharged into the needle assembly 30 via the second conduit P2 as the rotating part 2130 receives a driving force from the driving part 2110 and rotates.

Referring to FIGS. 16 and 17, as the pressure-applying part 2170 according to an embodiment of the present disclosure moves in the direction of the first axis AX1, the locking part 2190, specifically the locking body 2193, in contact with the protrusion 2172 formed to protrude from the pressure-applying part 2170, can be moved in the direction of the second axis AX2 that forms a certain angle with the first axis AX1.

Referring to FIGS. 16 and 17, the locking body 2193 according to an embodiment of the present disclosure is movable on the locking base part 2191 fixed in position to the body part 10, and the locking body 2193 may be moved in the direction of the second axis AX2 due to the elastic restoring force of the elastic member 2197 that connects the locking body 2193 to the locking base part 2191.

Referring to FIG. 17, when the pressure-applying part 2170 moves by a preset movement distance d, the first inclined surface 2195 formed on the locking body 2193 is moved in the direction of the second axis AX2 while in contact with the second inclined surface 2173 formed on the protrusion 2172, which is provided on the pressure-applying part 2170, and the locking body 2193 is located on the first axis AX1, which is the movement path of the pressure-applying part 2170.

As a result, the pressure-applying part 2170 may move in the direction of the first axis AX1 and remain in the state of being in contact with and compressing the tube part 2150.

In other words, the tube part 2150 formed of a flexible material exhibits an elastic restoring force in the direction of increasing the internal cross-sectional area that became zero due to compression, and this elastic restoring force may prevent the pressure-applying part 2170 from being pushed.

In addition, since the internal cross-sectional area of the tube part 2150 remains zero at the pair of contact points H1 and H2, a set amount of medical liquid may flow within the tube part 2150 as the rotating part 2130 rotates, and may be discharged toward the user by passing via the second conduit P2 and the needle assembly 30 connected to the second conduit P2.

In the medical liquid injection device 2000 according to an embodiment of the present disclosure, the locking part 2190 can move along the second axis AX2 forming a certain angle with the first axis AX1, which is the axis of movement of the pressure-applying part 2170, and can be located on the movement path of the pressure-applying part 2170, so that the movement of the pressure-applying part 2170 can be blocked.

In addition, since the movement of the pressure-applying part 2170 is blocked, it is possible to maintain the state in which the pressure-applying part 2170 is in contact with and compresses the tube part 2150.

In addition, by maintaining the compressed state of the tube part 2150, the set amount of medical liquid can be consistently discharged through the needle assembly 30 from the interior of the tube part 2150.

In addition, the structure, in which the locking part 2190 moves linearly along the second axis AX2 forming a certain angle with the first axis AX1 so that the locking part 2190 is disposed on the movement path of the pressure-applying part 2170 moving in the direction of the first axis AX1, allows the interior space of the medical liquid injection device 2000 to be utilized efficiently and allows the structure that blocks the movement of the pressure-applying part 2170 to be simplified.

Hereinafter, the configuration, operating principle, and effects of a medical liquid injection device according to another embodiment of the present disclosure will be described.

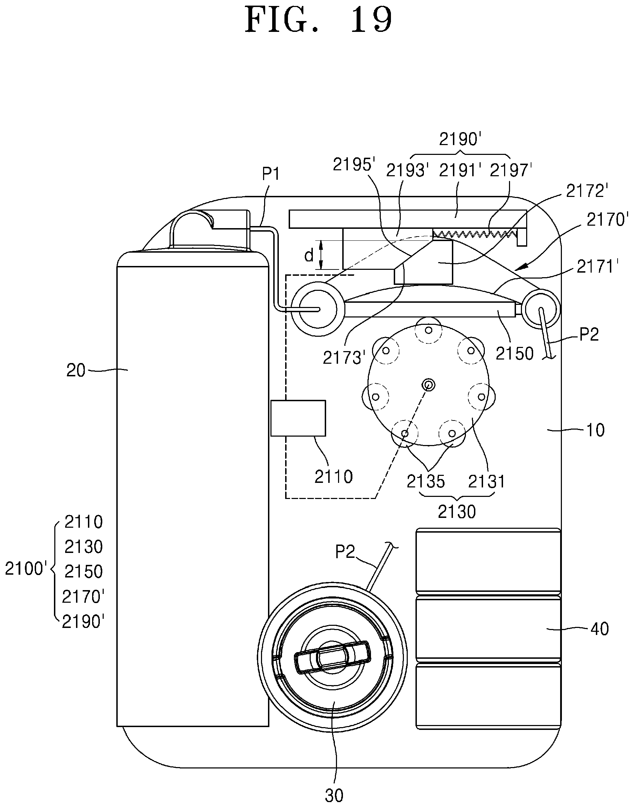

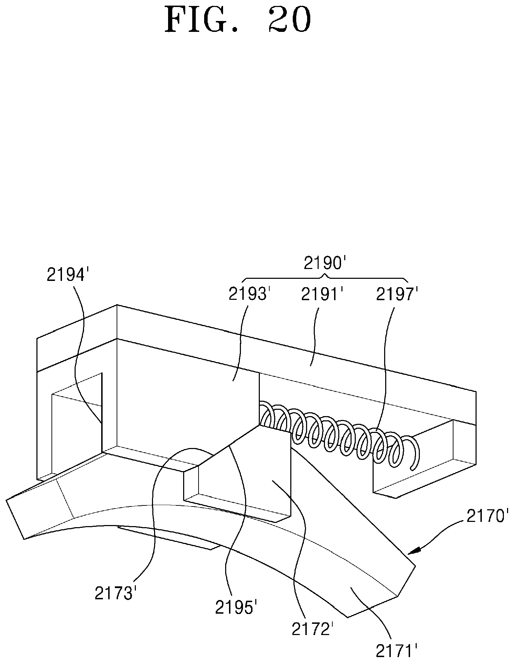

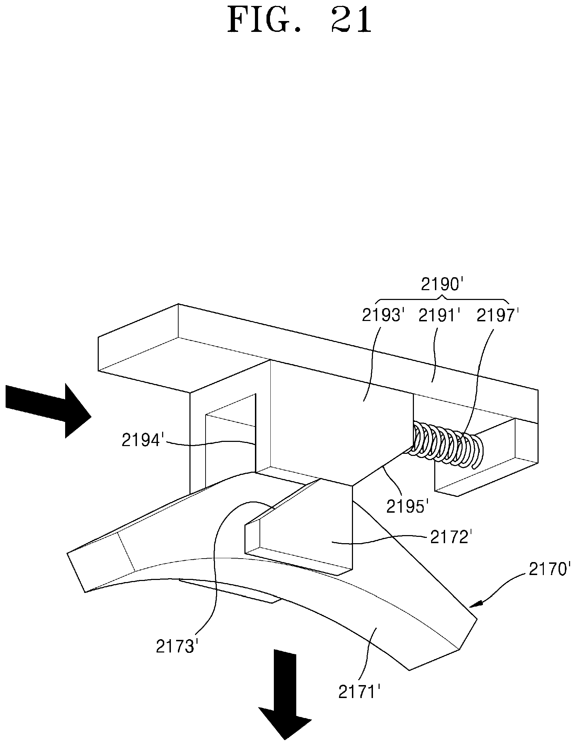

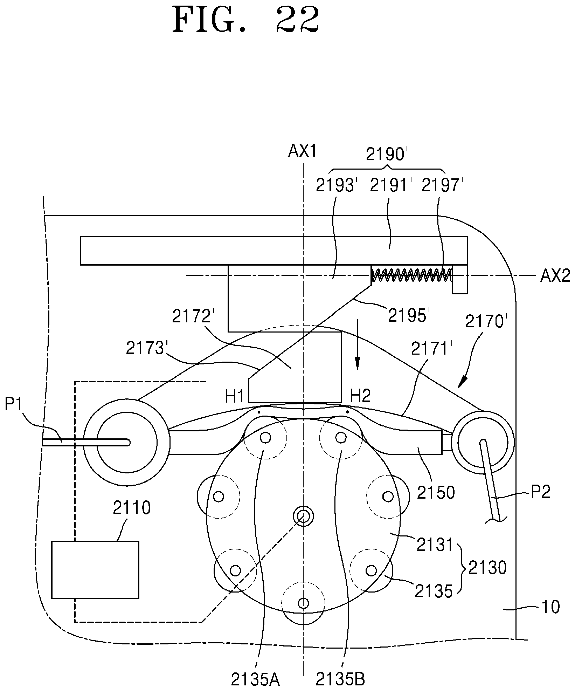

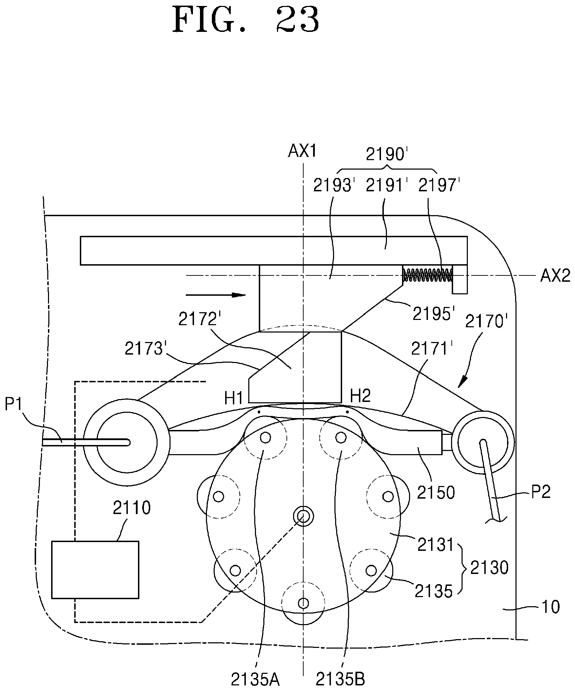

FIG. 19 is a plan view illustrating an interior of the medical liquid injection device according to another embodiment of the present disclosure. FIGS. 20 and 21 are perspective views schematically illustrating a locking part and a pressure-applying part according to another embodiment of the present disclosure. FIGS. 22 and 23 are enlarged views partially illustrating the locking part, a rotating part, and the pressure-applying part according to another embodiment of the present disclosure.

Referring to FIGS. 14 and 19 to 23, the medical liquid injection device according to another embodiment of the present disclosure is attached to a user, and may inject a preset amount of medical liquid stored therein to the user.

Referring to FIGS. 14 and 19 to 23, the medical liquid injection device according to another embodiment of the present disclosure may include a housing 5, an attachment part 6, a body part 10, a medical liquid dispensing assembly 2100', and a reservoir 20, a needle assembly 30, and a battery 40.

The medical liquid injection device according to another embodiment of the present disclosure is different from the medical liquid injection device according to an embodiment of the present disclosure in the configuration of the medical liquid dispensing assembly 2100', specifically a pressure-applying part 2170' and a locking part 2190', and thus, the following description will focus on the configuration, operating principle, and effects of the pressure-applying part 2170' and the locking part 2190'.

Referring to FIGS. 19 to 23, the pressure-applying part 2170' according to another embodiment of the present disclosure is disposed to face the rotating part 2130 with the tube part 2150 interposed therebetween and movable in the direction of the first axis AX1, and the pressure-applying part 2170' may, in conjunction with the rotating part 2130, come into contact with and apply pressure to the tube part 2150 from both sides.

Referring to FIGS. 19, 22, and 23, the tube part 2150 may be connected to the pressure-applying part 2170' according to another embodiment of the present disclosure, and specifically, both end portions of the tube part 2150 may be connected to a plurality of points of the pressure-applying part 2170'.

Referring to FIGS. 19, 22, and 23, since both end portions of the tube part 2150 are connected to a plurality of points of the pressure-applying part 2170', the tube part 2150 is disposed in a straight shape when not in contact with the contact part 2135, but may form a curved section when in contact with the contact part 2135 and in contact with and compressed by the pressure-applying part 2170' and the rotating part 2130.

Referring to FIGS. 19, 20, and 22, a curved surface part 2171' may be formed on one surface, specifically a lower surface (based on FIG. 19) of the pressure-applying part 2170', which faces the tube part 2150, to have a certain curvature to correspond to the curved section.

As a result, it is possible to correspond to the curved section formed in the tube part 2150 that is in contact with and compressed by the pressure-applying part 2170' and the rotating part 2130, and the tube part 2150 may stably remain in the compressed state.

Referring to FIGS. 19, 20, and 22, a preset region of the pressure-applying part 2170' according to another embodiment of the present disclosure may be formed in a relatively wide width, and may have a protrusion 2172' formed to protrude in an outward direction.

Referring to FIGS. 20 and 21, the pressure-applying part 2170' according to another embodiment of the present disclosure may be divided into a first region in which the protrusion 2172' is formed, and a second region in which the protrusion 2172' is not formed, and the second region in which the protrusion 2172' formed to protrude is not provided may be inserted into a guide part 2194' formed in the shape of a groove in a locking body 2193', and can be located on an inner side of the guide part 2194'.

Specifically, an inner surface of the guide part 2194' formed in the locking body 2193' may be in surface contact with and an outer surface of the pressure-applying part 2170', and the locking body 2193' may be moved in the direction of the second axis AX2 on the pressure-applying part 2170'.

The protrusion 2172' according to another embodiment of the present disclosure may have a second inclined surface 2173' formed on one side thereof that in contact with the locking part 2190', specifically the locking body 2193'. The second inclined surface 2173' formed on the protrusion 2172' is in contact with a first inclined surface 2195' formed on the locking body 2193', and may be formed in a shape corresponding to the shape of the first inclined surface 2195'.

Since the second inclined surface 2173' formed on the protrusion 2172' and the first inclined surface 2195' formed on the locking body 2193' are in contact with each other, the locking body 2193' may move along the second inclined surface 2173' formed on the protrusion 2172', and as the pressure-applying part 2170' moves in the direction of the first axis AX1, the locking body 2193' may stably move in the direction of the second axis AX2 that forms a predetermined angle with the first axis AX1.

Referring to FIGS. 19, 22, and 23, the pressure-applying part 2170' according to another embodiment of the present disclosure is movable in a preset direction, specifically, in the direction of the first axis AX1 upon receiving a driving force from the driving part 2110.

However, the present disclosure is not limited thereto, and the pressure-applying part 2170' is directly or indirectly connected to the rotating part 2130, and when the rotating part 2130 rotates in one direction upon receiving a driving force from the driving part 2110, in conjunction with the rotation of the rotating part 2130, the pressure-applying part 2170' may move in the direction of the first axis AX1 by a preset movement distance.

In the present disclosure, the term "movement distance" refers to a vertical distance d formed along the first axis AX1 with the second inclined surface 2173' as the hypotenuse, and as the pressure-applying part 2170' is moved closer to the rotating part 2130 by the movement distance, a curved section may be formed in the tube part 2150 disposed between the pressure-applying part 2170' and the rotating part 2130, and the tube part 2150 can come into contact with the rotating part 2130, specifically the contact part 2135 and may be compressed by the contact part 2135 and the curved surface part 2171' formed on the pressure-applying part 2170'.

Referring to FIGS. 22 and 23, the tube part 2150 is in contact with and compressed at the pair of contact points H1 and H2 by the pressure-applying part 2170' and the rotating part 2130, specifically, a plurality of contact parts 2135, and thus, may have a zero internal cross-sectional area.

As a result, a set amount of medical liquid may be accommodated inside the tube part 2150 between the plurality of points H1 and H2 at which the tube part 2150 is compressed by the pressure-applying part 2170' and the contact part 2135, and as the rotating part 2130 receives a driving force from the driving part 2110 and rotates, the set amount of medical liquid may flow within the tube part 2150 and may be discharged into the needle assembly 30 via the second conduit.

The pressure-applying part 2170' according to another embodiment of the present disclosure has the same configuration, operating principle, and effects as the pressure-applying part 2170 according to an embodiment of the present disclosure, except that the protrusion 2172'is formed to protrude outward on the outer side surface of the pressure-applying part 2170', rather than protruding toward the locking part 2190', and thus detailed descriptions of these components are omitted to the extent of overlapping.

Referring to FIGS. 20 and 21, the locking part 2190' according to another embodiment of the present disclosure is disposed to be in contact with the pressure-applying part 2170', is movable in the direction of the second axis AX2 that forms a certain angle with the first axis AX1, and can be located on a movement path of the pressure-applying part 2170'.

Referring to FIGS. 19 to 23, the locking part 2190' according to another embodiment of the present disclosure may include a locking base part 2191', the locking body 2193', and an elastic member 2197'.

Referring to FIGS. 20 and 21, the locking body 2193' according to another embodiment of the present disclosure has a preset width and depth, and may have the guide part 2194' formed in the shape of a groove.

Referring to FIGS. 20 and 21, the preset region of the pressure-applying part 2170' may be inserted in the inner side of the guide part 2194' formed in the locking body 2193'. Specifically, the second region of the pressure-applying part 2170' excluding the first region in which the protrusion 2172' is formed may be partially inserted into the inner side of the guide part 2194'.

Referring to FIGS. 21 to 23, sine the inner surface of the guide part 2194' formed in the locking body 2193' according to another embodiment of the present disclosure can come into surface contact with the outer surface of the pressure-applying part 2170', the locking body 2193' can move stably in the direction of the second axis AX2 while in contact with the pressure-applying part 2170'.

In addition, referring to FIG. 23, since the locking body 2193' is disposed on the first axis AX1, which is a movement path of the pressure-applying part 2170', specifically, the protrusion 2172', and comes into contact with and supports the pressure-applying part 2170' due to an elastic restoring force of the elastic member 2197', the pressure-applying part 2170' may be prevented from moving to an initial position before being moved due to an elastic restoring force of the compressed tube part 2150.

The locking part 2190' according to another embodiment of the present disclosure is the same as the locking part 2190 according to an embodiment of the present disclosure in the configuration, operating principle, and effects of the locking base part 2191 and the elastic member 2197, except that the guide part 2194' having a preset width and depth is formed in the shape of a groove so that the pressure-applying part 2170' can be partially inserted into the inner side of the locking body 2193', and thus detailed descriptions of these components are omitted to the extent of overlapping.

Referring to FIGS. 19 to 23, the medical liquid dispensing assembly 2100' according to another embodiment of the present disclosure is the same as the medical liquid discharge assembly 2100 according to an embodiment of the present disclosure in the configuration, operating principle, and effects of the driving part 2110, the rotating part 2130, the tube part 2150, and the control part 2160, except that the guide part 2194' is formed in the shape of a groove on the locking body 2193', a preset region of the pressure-applying part 2170', in which the protrusion 2172' is not formed, can be disposed on the inner side of the guide part 2194', and the inner surface of the guide part 2194' formed on the locking body 2193' is stably movable in the direction of the second axis AX2 while in contact with the outer surface of the pressure-applying part 2170', and thus detailed descriptions of these components are omitted to the extent of overlapping.

It should be noted that the spirit of the present disclosure is not limited to the embodiments described above, and not only the claims to be described later, but also all ranges equivalent to or equivalently changed from the claims fall within the scope of the spirit of the present disclosure.

### Industrial Applicability

According to the present disclosure, a medical liquid injection device is provided. In addition, the embodiments of the present disclosure may be applied to a medical liquid injection device that is industrially appliable and is capable of injecting a medical liquid into the body of a user, and the like.

## Claims

1. A medical liquid discharge assembly comprising:
a driving part configured to move in a preset direction;
a rotating part configured to come into contact with the driving part, and to rotate in one direction in response to a movement of the driving part; a tube part disposed adjacent to the rotating part and including a flexible material;
a pressure-applying part configured to come into contact with the rotating part, and to come into contact with and apply pressure to the tube part in response to a rotation of the rotating part; and
a locking part configured to move in a preset direction, and to fix a position of the pressure-applying part by coming into contact with and supporting one surface of the pressure-applying part while the pressure-applying part applies pressure to the tube part.

2. The medical liquid discharge assembly of claim 1, wherein
the pressure-applying part applies pressure to the tube part so that the tube part is compressed at a contact point at which the pressure-applying part comes into contact with the tube part.

3. The medical liquid discharge assembly of claim 1, wherein
at least a portion of the tube part has a curved section extending in a circumferential direction thereof.

4. The medical liquid discharge assembly of claim 1, wherein
the pressure-applying part rotates while applying pressure to the tube part.

5. The medical liquid discharge assembly of claim 1, wherein
the pressure-applying part includes:
a pressure-applying body configured to move in a preset direction on the rotating part and to come into contact with and apply pressure to the tube part; and
a pushing part disposed between the pressure-applying body and the rotating part, and configured to push the pressure-applying body in such a direction that the pressure-applying body is spaced apart from the rotating part.

6. The medical liquid discharge assembly of claim 5, wherein
the pushing part is provided as a plurality of pushing parts.

7. The medical liquid discharge assembly of claim 5, wherein
the pushing part exhibits an elastic restoring force in such a direction that the pushing part is away from the rotating part.

8. The medical liquid discharge assembly of claim 1, wherein
the locking part includes an elastically deformable material.

9. The medical liquid discharge assembly of claim 8, wherein
one end portion of the locking part exhibits an elastic restoring force in a direction toward a longitudinal central axis of the locking part.

10. A medical liquid injection device comprising:
a needle assembly;
a reservoir configured to store a medical liquid to be discharged to the needle assembly;
a driving part configured to move in a preset direction;
a rotating part configured to come into contact with the driving part, and to rotate in one direction in response to a movement of the driving part; a tube part disposed between the needle assembly and the reservoir and including a flexible material;
a pressure-applying part configured to come into contact with the rotating part, and to come into contact with and apply pressure to the tube part in response to a rotation of the rotating part; and
a locking part configured to move in a preset direction, and to fix a position of the pressure-applying part by coming into contact with and supporting one surface of the pressure-applying part while the pressure-applying part applies pressure to the tube part.
